(19) Europäisches Patentamt
European Patent Office
Office européen des brevets

(11) EP 4 696 337 A1

(12) EUROPEAN PATENT APPLICATION

(43) Date of publication:
**18.02.2026 Bulletin 2026/08**

(21) Application number: **24194818.1**

(22) Date of filing: **15.08.2024**

(51) International Patent Classification (IPC):
***A61L 27/22** (2006.01)* ***A61L 27/26** (2006.01)*
***A61L 27/38** (2006.01)* ***A61L 27/52** (2006.01)*
***B33Y 70/00** (2020.01)* ***C08L 89/06** (2006.01)*

(52) Cooperative Patent Classification (CPC):
**A61L 27/222; A61L 27/26; A61L 27/38; A61L 27/52; B33Y 70/00; C08J 3/075; C08L 89/06;** C12N 2533/54

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **EMPA**
**8600 Dübendorf (CH)**

(72) Inventors:
- **HAMMER, Tobias**
  **8600 Dübendorf (CH)**
- **WEI, Kongchang**
  **8600 Dübendorf (CH)**
- **ROSSI, René**
  **8600 Dübendorf (CH)**
- **MANIURA-WEBER, Katharina**
  **8600 Dübendorf (CH)**
- **YANG, ke**
  **8600 Dübendorf (CH)**
- **ROTTMAR, Markus**
  **8600 Dübendorf (CH)**
- **SPIRIG, Tobias**
  **8600 Dübendorf (CH)**

(74) Representative: **Schulz Junghans Patentanwälte PartGmbB**
**Großbeerenstraße 71**
**10963 Berlin (DE)**

(54) **FISH GELATINE BASED HYDROGEL FORMULATION**

(57) The invention relates to an aqueous composition comprising thiolated and norbornene-modified cold-water species-derived gelatine. A further aspect of the invention relates to a method to generate a three-dimensional hydrogel object, particularly an in vitro model for disease modelling/drug testing or an implant, comprising providing a composition according to the invention and forming the hydrogel object by bioprinting (extrusion-based or digital light-based (DLP)) or molding/casting to provide the three-dimensional hydrogel object.

Also provided are a hydrogel implant obtained by a method according to the invention, and a kit comprising dried thiolated and norbornene-modified cold-water marine species gelatine.

EP 4 696 337 A1

## Description

### Field

The present invention relates to gelatine compositions comprising two modified cold-water marine species derived gelatine polymers that are able to photo-crosslink with one another. The invention further encompasses methods and uses of the compositions, particularly as an in vitro model for disease modelling/drug testing.

### Background

Hydrogels are defined as polymeric networks capable of retaining large amounts of water, and have been widely employed in the rapidly growing field of tissue engineering due to their characteristics being reminiscent of natural tissue. Hydrogels promise to provide candidates for the clinical application of cell-loaded scaffolds. They are highly tuneable, making it possible to equip them with a variety of chemical modifications to alter their mechanical properties, degradability, biocompatibility or swelling behaviour. Hydrogels can be composed of a wide selection of natural or synthetic polymers, with the former being particularly suitable for biomedical applications due to their inherent biocompatibility and cell-instructive environment (e.g. RGD-sites).

Natural biopolymer-based hydrogels are mainly formed from polysaccharides (e.g. chitosan, cellulose, alginate) or proteins/polypeptides (e.g. collagen, gelatine, fibroin). Chemical modification of such biopolymers is usually needed to allow for inter-polymer cross-linking, which leads to the formation of stable hydrogels [Muir etal., Chemical reviews 121.18 (2020): 10908-10949]. Gelatine, which is obtained from the hydrolytic degradation of collagen, is one of the most widely used biopolymers for the fabrication of hydrogels for biomedical applications, since it shares many of collagen's important chemical characteristics [Yoon et al. PloS one 11.10 (2016): e0163902; Bellis, Biomaterials 32.18 (2011): 4205-4210]. Commonly, the gelatine used is obtained from mammalian sources, such as porcine or bovine. Recently, gelatine form cold-water fish has experienced a rise in interest due to its lower melting and gelling point compared to its mammalian counterparts, making it far easier to dissolve and maintain in a liquid state at moderate temperatures, a trait that makes the material attractive for certain biofabrication processes (e.g. bioprinting) [Le Thi et al., Journal of Biomaterials Applications 34.9 (2020): 1216-1226; Nitsuwat et al. Lwt 141 (2021): 110899]. Furthermore, cold-water fish gelatine does not face the same cultural or religious restrictions in its consumption or incorporation as would mammalian gelatine, and there are no reported risks of zoonotic diseases from its use. This is particularly relevant for materials with intended applications as medical implants or grafts.

One issue affecting the structural features and mechanical stability of as-prepared biopolymeric hydrogels is their swelling in aqueous media [Zhan et al., Materials Science and Engineering: C 127 (2021): 112208]. Apart from the deterioration of mechanical properties, the swelling-induced volume expansion of hydrogel constructs can, among other problems, compromise the accuracy of fabricated structures, or result in the oppression or damaging of surrounding tissues in in vivo applications.

Preventing the swelling of natural biopolymeric hydrogels usually requires substantial chemical modification, which usually interferes with its biocompatibility and use as a cell-culture platform.

CN112220963B discloses a non-swellable, UV-crosslinkable chitosan hydrogel, which displays non- swelling behaviour in a range of media. However, the lack of cell-adhesive and cell-degradation functionalities in chitosan polymers makes them less suitable than gelatin-based hydrogels for biomedical applications.

WO2021180795A1 discloses dynamic covalent hydrogels crosslinked by phenylboronic acid and glucamine.

WO2024113021A1 discloses fish gelatine hydrogels crosslinked by tetravalent PEG linkers.

Based on the above-mentioned state of the art, the objective of the present invention is to provide means and methods to provide improved fish-gelatine based hydrogel compositions and methods of their use, as well as improved implants obtainable by the compositions and methods of the invention. This objective is attained by the subject-matter of the independent claims of the present specification, with further advantageous embodiments described in the dependent claims, examples, figures and general description of this specification.

### Summary of the Invention

The present invention provides the advantages of a biopolymeric material (cold-water fish gelatine) while also demonstrating rapid gelation properties due to a UV-mediated, click-reaction-induced crosslinking mechanism, resulting in hydrogels with tuneable mechanical and structural stability, as well as controllable swelling behaviours. Such fully cold-water fish gelatine-based hydrogels with excellent cytocompatibility and low immunogenicity are promising for various in vitro and in vivo biomedical applications, including but not limited to 2D/3D in vitro cell culture, 3D bioprinting, in vivo tissue regeneration. The gels prepared according to the invention demonstrate non- swelling properties in physiological media such as PBS and cell culture media (DMEM) and even strongly diluted PBS (0.125X)

A first aspect of the invention relates to an aqueous composition comprising:

a. thiolated cold-water fish or cold-water marine fauna-derived gelatine and

b. norbornene-modified or vinyl-ester modified cold-water fish or cold-water marine fauna-derived gelatine.

A further aspect of the invention relates to a method to generate a three-dimensional hydrogel object, particularly an in vitro model for disease modelling/drug testing or an implant, comprising the steps of:

a. providing a composition according to any one of the aspects or embodiments of the invention recited herein;

b. forming the hydrogel object by bioprinting (extrusion-based or digital light-based (DLP)) or molding/casting to provide the three-dimensional hydrogel object.

Another aspect of the invention relates to a hydrogel implant obtained by a method according to the invention.
Also provided is a kit comprising dried thiolated cold-water marine species, particularly cold-water fish gelatine and norbornene-modified cold-water marine species, particularly cold-water fish gelatine.

*Terms and definitions*

*General*

For purposes of interpreting this specification, the following definitions will apply and whenever appropriate, terms used in the singular will also include the plural and vice versa. In the event that any definition set forth below conflicts with any document incorporated herein by reference, the definition set forth shall control.
The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."
Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.
Reference to "about" a value or parameter herein includes (and describes) variations that are directed to that value or parameter per se. For example, description referring to "about X" includes description of "X."
As used herein, including in the appended claims, the singular forms "a", "or" and "the" include plural referents unless the context clearly dictates otherwise.
"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).
Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.
The term *thiolated* in the context of the present specification, when applied to gelatin, relates to gelatin being modified to bear a thiol function. Proteins can be thiolated by modifying reactive side chains with thiol-bearing moieties. Amine bearing side chains, for example lysin, may be modified by reaction of the protein with an activated (e.g., by carbodiimide activation) thiol-bearing carboxylic acid, such as N-acetylcysteine, or N-acetylhomocystein thiolactone.
The term *PBS* in the context of the present specification relates to Phosphate Buffered Saline: NaCl: 8.0 g/L; KCl: 0.2 g/L; $Na_2HPO_4$: 1.44 g/L $KH_2PO_4$: 0.24 g/L, pH 7.4.

The term *photoinitiator* in the context of the present specification relates to any compound capable of initiating a polymerization reaction when triggered by electromagnetic radiation, particularly by light in the visible or near infrared or near UV spectrum. Non-limiting examples of photoinitiators useful to practice the current invention include single photon initiators such as Li-phenyl-2,4,6trimethylbenzoylphosphinate, 2-hydroxy-4'-(2-hydroxyethoxy)-2-methylpropiophenone (Irgacure 2959), Eosin Yellow + triethanol amine, bisacylphosphineoxide (BAPO) salts such as BAPO-ONa and BAPO-OLi; TPO (diphenyl(2,4,6-trimethylbenzoyl)phosphine oxide) lithium or sodium, VA-086 (2,2'-azobis[2-methyl-N-(2-hydroxyethyl)propionamide]; CAS No. 61551-69-7), and two-photon initiators exemplified by P2CK (3,3'-((((1E,1'E)-(2-oxocyclopentane-1,3-diylidene)bis(methanylylidene))bis(4,1-phenylene))bis(methylazanediyl))dipropanoate), G2CK (sodium 2,2'-((((1E,1'E)-(5-methyl-2-oxocyclohexane-1,3-diylidene)bis(methanylylidene))bis(4,1-phenylene))bis(methylazanediyl))diacetate), DAS (tetrapotassium 4,4'-(1,2-ethenediyl)bis[2-(3-sulfophenyl)diazenesulfonate]).

See also Benedikt et al., J. Polymer Science Part A Polymer Chemistry (2015) Vol. 54, 473-479; https://doi.orq/10.1002/pola.27903 and Tromayer et al., Polymer Chemistry (2018) 9, 3108-3117; https://doi.org/10.1039/C8PY00278A.

Any patent document cited herein shall be deemed incorporated by reference herein in its entirety.

Detailed Description of the Invention

A first aspect of the invention relates to an aqueous composition comprising:

a. thiolated cold-water fish or cold-water marine fauna-derived gelatine and

b. norbornene-modified or vinyl-ester modified cold-water fish or cold-water marine fauna-derived gelatine.

*The gelatine gel point is lower than mammalian gelatine*

Importantly, the gelatine is characterized by a gel point of ≤20°C in unmodified form. The modified gelatine is expected to be similar in gelling and melting behaviour compared to the precursor, unmodified gelatine. In certain embodiments, the gelatine is characterized by a gel point of ≤15°C. In certain embodiments, the gelatine is characterized by a gel point of ≤13°C. In certain embodiments, the gelatine is characterized by a gel point of ≤12°C. In certain embodiments, the gelatine is characterized by a gel point of ≤10°C.

In certain embodiments, the gelatine is characterized by a gel point of ≥4°C and ≤20°C. In particular embodiments, the gelatine is characterized by a gel point of ≥4°C and ≤15°C. In certain embodiments, the gelatine is characterized by a gel point of ≥4°C and ≤13°C. In certain embodiments, the gelatine is characterized by a gel point of ≤12°C. In certain embodiments, the gelatine is characterized by a gel point of ≥4°C and ≤10°C.

The gel point for the purpose of definitions given in the present specification, is determined by small-strain oscillatory rheology measurement, wherein 25 mm plain plate geometry with 1 mm gap is used for loading 5 wt% gelatin solution at 50°C. Time-sweep (wit ≥4°C and h 1 % shear strain and 1 Hz frequency) is carried out with temperature ramping from 50°C to 5°C (temperature gradient 0.5°C/min). The crossover temperature point when storage modulus (G') equals to loss modulus (G") is determined as the gel point.

In certain embodiments, the gelatine is obtained from a marine species adapted to ambient water temperatures <15°C, particularly from a marine species adapted to ambient water temperatures <10°C.

In certain embodiments, the gelatine is cold water fish gelatine.

In certain embodiments, the gelatine is obtained from a fish species selected from the group of *Salmo, Gadus, Oncorhynchus* and *Merluccius.* In certain embodiments, the gelatine is obtained from cold-water jellyfish.

*The content of modifications*

In particular embodiments, the thiolated gelatine is characterized by a content of between 50 and 500 μmol thiol groups per gram of gelatine. In more particular embodiments, the thiolated gelatine is characterized by a content of between 100 and 300 pmol thiol groups per gram of gelatine.

In particular embodiments, the number of proline residues in the thiolated and norbornene-modified gelatin is not more than 20% of the total number of amino acid residues in the gelatin.

A large number of so-called click-chemistry reactions are available to the skilled artisan to quickly, simply and selectively introduce the thiol and ene modifications, respectively, that facilitate crosslinking.

If activated esters are used to introduce modifications, both thiol modification and norbornene modification in the respective gelatine fraction will mainly be made on lysine side chains. Thiolation is readily available by activated (carbodiimide) acetylcystein (ACC) and homocysteinethiolactone functionalization. One example is depicted in the following structure:

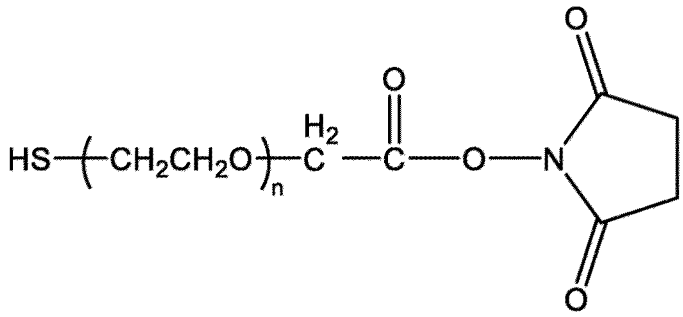

An example of a norbornene containing compound useful for practicing the invention in the following structure:

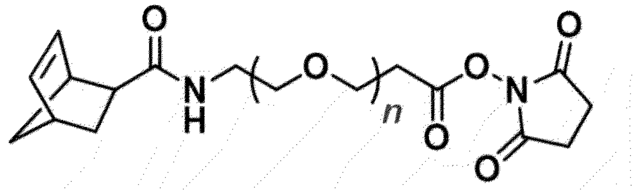

Advantages of thiol-norbornen photopolymerization in hydrogel crosslinking include the fact that the resulting gels are cytocompatible, and that the polymerization reaction is controllable both spatially and temporally; these advantages are not nearly as available for base-catalyzed Michael-type addition reactions between thiols and conjugated, unsaturated functional groups (e.g., maleimide, acrylate, methacrylate and vinyl sulfone).

Alternatively, the ene bearing moiety employed as partner for thiol-ene click crosslinking is, instead of norbornene, vinyl ester.

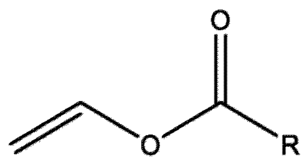

with R being the gelatine backbone, optionally connected through a short linker.

Similarly, it is possible to modify the polymer through DCCI-activation of the gelatine and subsequent reaction with a thiol bearing amine. In other words, Asp and Glu residues of gelatine can be activated and then react with thiol bearing amine; however, this reaction needs to be calibrated carefully to avoid cross-reaction with lysine moieties in the gelatine.

In particular embodiments, the thiolated gelatine is characterized by between 50 and 500 pmol norbornene groups per gram of gelatine. In more particular embodiments, the thiolated gelatine is characterized by between 100 and 300 pmol norbornene groups per gram of gelatine.

In certain embodiments, norbornene modification is effected through activated norbornene acetic acid, or norbornene diacetic acid. Another example is the norbornene acetic acid linke to activated ester through a PEG linker. The inventors used the activated norbornene acetic acid in order to minimize the content of any synthetic component in the formation of the gels so as to not interfere with biological components such as cells.

*Concentration of gelatine in the composition*

In certain embodiments, the total concentration of gelatine ranges from 3 to 20 % (w/w).

Gels in the general range of 3 to 10% gelatine (w/w; all concentrations given for gelatine herein are deemed to be in w/w unless specified otherwise) are useful for low-modulus materials, such as may be required for injectable formulations of cell-containing gels.

In certain embodiments, the total concentration of gelatine ranges from 3% to 8%. In certain embodiments, the total concentration of gelatine ranges from 3.5% to 8%. In certain embodiments, the total concentration of gelatine ranges from 4% to 8%. In certain embodiments, the total concentration of gelatine ranges from 5% to 8%.

In certain embodiments, the total concentration of gelatine ranges from 3% to 10%. In certain embodiments, the total concentration of gelatine ranges from 3.5% to 10%. In certain embodiments, the total concentration of gelatine ranges from 4% to 10%. In certain embodiments, the total concentration of gelatine ranges from 5% to 10%.

Gels in the general range of 3 to 15% gelatine (w/w) are useful for medium-modulus materials, such as may be required for direct encapsulation of cells.

In certain embodiments, the total concentration of gelatine ranges from 3% to 15%. In certain embodiments, the total concentration of gelatine ranges from 3.5% to 15%. In certain embodiments, the total concentration of gelatine ranges from 4% to 15%. In certain embodiments, the total concentration of gelatine ranges from 5% to 15%.

Gels in the general range of 3 to 20% gelatine (w/w) are useful for high-modulus materials, such as may be useful for 3D printing.

In certain embodiments, the total concentration of gelatine ranges from 3% to 20%. In certain embodiments, the total concentration of gelatine ranges from 5% to 20%. In certain embodiments, the total concentration of gelatine ranges from 7% to 20%. In certain embodiments, the total concentration of gelatine ranges from 10% to 20%. In certain embodiments, the total concentration of gelatine ranges from 12% to 20%. In certain embodiments, the total concentration of gelatine ranges from 15% to 20%. In certain embodiments, the total concentration of gelatine ranges from 17% to 20%.

*The ratio of thiol to norbornene*

The inventors have found that if the gelatine composition comprises predominantly norbornene modified material, the solution will not gel (e.g. 1:4 thiol:norbornene will not form gels). As for thiol-dominant ratios, gel formation still takes place at higher ratios (e.g. 4:1 thiol:norbornene) though the modulus of the gels gets increasingly lower. Ratios closer to equal amounts (2:3, 1:1, 3:2, 7:3) are more likely to be practiced.

In certain embodiments, the ratio of thiolated gelatine to norbornene-modified gelatine ranges from 1:3 to 4:1.

In certain embodiments, the ratio of thiolated gelatine to norbornene-modified gelatine ranges from 2:3 to 3:2.

In certain embodiments, the ratio of thiolated gelatine to norbornene-modified gelatine ranges from 1:4 to 3:2. In certain embodiments, the ratio of thiolated gelatine to norbornene-modified gelatine ranges from 2:3 to 4:1.

In certain embodiments, the ratio of thiolated gelatine to norbornene-modified gelatine ranges from 1:4 to 1:1.2. In certain embodiments, the ratio of thiolated gelatine to norbornene-modified gelatine ranges from 1.2:1 to 4:1.

The aqueous composition according to claim 7, wherein the ratio of thiolated gelatine to norbornene-modified gelatine ranges from 1:1,2 to 1,2:1.

In certain embodiments, the ratio of thiol groups to norbornene groups in the composition ranges from 2:3 to 3:2.

In certain embodiments, the composition consists of thiolated gelatine and norbornene-modified gelatine and an aqueous solution of electrolytes, characterized by a pH ranging from 5.5 to 8.0 and an osmolality of: 310 - 350 mOsm/Kg $H_2O$.

In certain embodiments, the electrolytes do not comprise calcium ions / wherein the composition does not comprise $\geq 100 \mu mol/L\ Ca^{2+}$ ions.

In certain embodiments, the aqueous composition according to the invention further comprises a photoinitiator, making the composition ready to use in 3D printing applications.

Non-limiting examples of photoinitiators useful to practice the current invention include single photon initiators such as Li-phenyl-2,4,6trimethylbenzoylphosphinate (LAP), 2-hydroxy-4'-(2-hydroxyethoxy)-2-methylpropiophenone (Irgacure 2959), Eosin Yellow + triethanol amine, bisacylphosphineoxide (BAPO) salts such as BAPO-ONa and BAPO-OLi; TPO (diphenyl(2,4,6-trimethylbenzoyl)phosphine oxide) lithium or sodium, VA-086 (2,2'-azobis[2-methyl-N-(2-hydroxyethyl)propionamide]; CAS No. 61551-69-7), and two-photon initiators exemplified by P2CK (3,3'-((((1E,1'E)-(2-oxocyclopentane-1,3-diylidene)bis(methanylylidene))bis(4,1-phenylene))bis(methylazanediyl))dipropanoate), G2CK (sodium 2,2'-((((1E,1'E)-(5-methyl-2-oxocyclohexane-1,3-diylidene)bis(methanylylidene))bis(4,1-phenylene))bis(methylazanediyl))diacetate), DAS (tetrapotassium 4,4'-(1,2-ethenediyl)bis[2-(3-sulfophenyl)diazenesulfonate]).

In certain embodiments, the aqueous composition according to the invention further comprises live mammalian cells.

As the material can be used to reconstruct various tissues, there is a wide range of cells that can be employed advantageously in practicing aspects of the invention that make use of cells.

In particular embodiments, the mammalian cells are selected from the group consisting of skin fibroblasts, induced pluripotent stem cells (iPSC); macrophages, T cells; endothelial cells (EC).

In certain embodiments, the compositions provided herein consist of the two types of modified (thiolated and ene-modified) gelatine components in aqueous, optionally buffered solution in presence or absence of live mammalian cells.

In other embodiments, a range of possible additives might be added if parameters such as viscosity of the compositions provided herein are to be further modified to accommodate a specific use, such as 3D printing. In some embodiments, for example, nanofiber fragments may be used to increase the viscosity of inks for 3D printing. The inventors employed viscous support baths (FRESH method: Shiwarski et al., APL Bioeng. 2021 Mar; 5(1): 010904). There is also the possibility to include other components such as nanoparticles, growth factors to stimulate cell growth, proliferation and differentiation, or antibiotics. Other possible additives include extracellular vesicles, ECM-derived

biopolymers (polysaccharides), and plasmids.

A further aspect of the invention relates to a method to generate a three-dimensional hydrogel object, particularly an in vitro model for disease modelling/drug testing or an implant, comprising the steps of:

a. providing a composition according to any one of the aspects or embodiments of the invention recited herein;
b. forming the hydrogel object by bioprinting (extrusion-based or digital light-based (DLP)) or molding/casting to provide the three-dimensional hydrogel object.

In particular embodiments, the composition is prepared in pure water or an isotonic sugar solution, and subsequent to step b, the hydrogel object is contacted with a membrane and placed in cell culture medium.

These embodiments facilitate the swelling/deswelling mediated adhesion of the hydrogel to a membrane component or another hydrogel surface. This may be achieved by first swelling the bulk hydrogel in distilled water or, for cytocompatible applications, an isotonic (0.25 M) sucrose solution, followed by the placement of the membrane atop the hydrogel and the subsequent de-swelling of the bulk hydrogel in PBS/DMEM. The strain exerted on the membrane through the de-swelling of the hydrogel causes the formation of wrinkles, which reinforce the adhesion between the two structures and impart the membrane with a distinct topography.

Another aspect of the invention relates to a hydrogel implant obtained by a method according to the invention.

Also provided is a kit comprising dried thiolated cold-water marine species, particularly cold-water fish gelatine and norbornene-modified cold-water marine species, particularly cold-water fish gelatine.

The invention further encompasses the following embodiments:

1. An aqueous composition comprising:

a. thiolated gelatine and

b. norbornene-modified or vinyl-ester modified gelatine

wherein the gelatine is characterized by a gel point of ≤20°C.

2. The aqueous composition according to embodiment 1, wherein the gelatine is characterized by a gel point of ≤15°C.

3. The aqueous composition according to embodiment 1, wherein the gelatine is characterized by a gel point of ≤ 13°C.

4. The aqueous composition according to any one of the preceding embodiments, wherein the thiolated gelatine is characterized by between 50 and 500 pmol thiol groups per gram of gelatine.

5. The aqueous composition according to any one of the preceding embodiments, wherein the thiolated gelatine is characterized by between 100 and 300 pmol thiol groups per gram of gelatine.

6. The aqueous composition according to any one of the preceding embodiments, wherein the number of proline residues in the gelatine is not more than 20% of the total number of amino acid residues in the gelatine.

7. The aqueous composition according to any one of the preceding embodiments, wherein the thiolated gelatine is characterized by between 50 and 500 pmol norbornene groups per gram of gelatine.

8. The aqueous composition according to any one of the preceding embodiments, wherein the thiolated gelatine is characterized by between 100 and 300 pmol norbornene groups per gram of gelatine.

9. The aqueous composition according to any one of the preceding embodiments, wherein the total concentration of gelatine ranges from 3 to 20 % (w/w).

10. The aqueous composition according to any one of the preceding embodiments, wherein the ratio of thiolated gelatine to norbornene-modified gelatine ranges from 1:3 to 4:1.

11. The aqueous composition according to embodiment 10, wherein the ratio of thiolated gelatine to norbornene-modified gelatine ranges from 2:3 to 3:2.

12. The aqueous composition according to embodiment 10, wherein the ratio of thiolated gelatine to norbornene-modified gelatine ranges from 1:1,2 to 1,2:1.

13. The aqueous composition according to any one of the preceding embodiments, wherein the ratio of thiol groups to norbornene groups in the composition ranges from 2:3 to 3:2.

14. The aqueous composition according to any one of the preceding embodiments, wherein the composition consists of thiolated gelatine and norbornene-modified gelatine and an aqueous solution of electrolytes, characterized by an osmolality of: 310 - 350 mOsm/Kg $H_2O$.

15. The aqueous composition according to embodiment 14, wherein the electrolytes do not comprise calcium ions / wherein the composition does not comprise $\geq 100 \mu mol/L$ $Ca^{2+}$ ions.

16. The aqueous composition according to any one of the preceding embodiments, wherein the gelatine is cold water fish gelatine.

17. The aqueous composition according to any one of the preceding embodiments, further comprising a photoinitiator.

18. The aqueous composition according to any one of the preceding embodiments, further comprising live mammalian cells.

19. The aqueous composition according to embodiment 18, wherein the mammalian cells are selected from the group consisting of skin fibroblasts, induced pluripotent stem cells (iPSC); macrophages, T cells; endothelial cells (EC).

20. A method to generate a three-dimensional hydrogel object, particularly an in vitro model for disease modelling/-drug testing or an implant, comprising the steps of:

a. providing a composition according to any one of the preceding embodiments 1 to 19;

b. forming the hydrogel object by bioprinting (extrusion-based or digital light-based (DLP)) or molding/casting to provide the three-dimensional hydrogel object.

21. The method according to embodiment 20, wherein the hydrogel object is incubated in pure water or an isotonic sugar solution, then contacted with a membrane and placed in salt-containing medium.
The de-swelling can be induced by salt-containing solutions, including PBS and cell culture medium.

22. A hydrogel implant obtained by a method according to embodiment 20 or 21.

23. A kit comprising dried thiolated cold-water species gelatine and norbornene-modified cold-water species gelatine.

Wherever alternatives for single separable features are laid out herein as "embodiments", it is to be understood that such alternatives may be combined freely to form discrete embodiments of the invention disclosed herein.
The invention is further illustrated by the following examples and figures, from which further embodiments and advantages can be drawn. These examples are meant to illustrate the invention but not to limit its scope.

*Description of the Figures*

Fig. 1 shows the design and features of a fully cold-water fish gelatine-based hydrogel (cfGel-Hydrogel).

Fig. 2 shows chemical structure and non-swelling property of fully cold-water fish gelatine-based hydrogels. (A) synthesis and "click" crosslinking of functional cold water fish gelatine polymers (cfGel-SH and cfGel-NB). (B) Oscillatory time-sweep photorheology shows fast curing of cfGel-Hydrogels (5% (w/w), 1:1 cfGel-NB:cfGel-SH) under UV 365 nm irradiation (10mW/cm2). The gray area indicates the timespan of UV exposure. n = 3. (C) Rheological properties of cfGel-Hydrogels with different R, n = 3. (D) Oscillatory strain-sweep rheological measurements of in-situ formed cfGel-Hydrogels. n = 3. (E) Representative rheological frequency-sweep measurement of 5wt% cfGel-Hydrogels (R = 5:5).

Fig. 3 shows swelling behavior of cfGel-Hydrogels. (A) Swelling ratio (SR) of cfGel-Hydrogels consisting of various polymer weight ratios between cfGel-NB and cfGel-SH (R) after 24 h, n = 3. (B) Swelling ratio of cfGel-Hydrogels (R = 5:5) with different total polymer concentrations after incubation in 37°C PBS for 24 h and 168 h, n = 3. (C) Degree of swelling of cfGel-Hydrogels in the presence of PBS at various concentrations compared to ultrapure water (UPW). (D) Comparison of the swelling ratio of each condition after 24 h. Inset shows the range from 8× to 0.5×, n = 3. (E) Swelling behavior of cfGel-Hydrogels in solutions of NaCl and CaCl2, and PBS n = 3. (F) Swelling of cfGel-Hydrogels in isotonic solutions of sucrose (0.25 M) in comparison to UPW and PBS, n = 3.

Fig. 4 shows cyclic compressive test of cfGel-Hydrogels in PBS (37 °C) (A) and UPW (B). Hysteresis is presented as mean ± s.d. n = 3. (C) Stress-strain curves for determining Young's modulus (inset) of cfGel-Hydrogels with linear fitting (stress values were adjusted for visualization purposes; APBS = 1000, AUPW = 0). ***P < 0.001 (D) Cyclic compressions of cfGel-Hydrogels in PBS (37 °C) under various residual strains ($\varepsilon$r).

Fig. 5 shows schematic illustration of the proposed molecular mechanism supporting the observed swelling and mechanical properties exhibited by cfGel-Hydrogels in either PBS or UPW.

Fig. 6 shows 3D cell encapsulation and cytocompatibility assessment. (A) Schematic representation of the encapsulation of cells in cfGel-Hydrogels with different total polymer concentrations (R = 5:5). (B) Metabolic activity of encapsulated HDFs at different time points as determined by Alamar Blue assay (n = 3, **P < 0.01, ***P < 0.001). (C) Live/dead images of encapsulated HDFs. Scale bars = 200 μm.

Fig. 7 shows cell-free and cell-inclusive 3D printing of cfGel-Hydrogels. (A) Post-printing images of rhodamine B-containing cell-free cfGel-Hydrogels directly after printing (suspended in agarose) and after incubation in DPBS (37 °C) for up to 2 weeks. Scale bars = 1 mm (B)Live/Dead and immunofluorescence images of cell-laden printed constructs. (C) Measured surface area of imaged grids at different time points. Agarose-suspended samples could not be measured properly and were therefore left out. n = 3

Fig. 8 shows 1H NMR of cfGel-NB functional polymer.

Fig. 9 shows rapid gelation of 5wt% cfGel-Hydrogels with different ratios (R, by weight) between cfGel-NB and cfGel-SH. Gray area denotes the timespan of UV-exposure.

Fig. 10 shows oscillatory strain sweep measurements of cfGel-Hydrogels (R = 5:5) at different total cfGel concentrations.

Fig. 11 shows shows oscillatory frequency sweep measurements of cfGel-Hydrogels (R = 5:5) at different total cfGel concentrations.

Fig. 12 shows detailed timeline of swelling ratio of cfGel-Hydrogels with varying polymer concentration (fixed R = 5:5) as measured by changes in weight. Total polymer concentration of cfGel-Hydrogel precursor formulations shows no significant effect on swelling properties of resultant gels. (n=3)

Fig. 13 shows cyclic compression test of cfGel-Hydrogel in PBS or ultrapure water (UPW). (A) Schematic illustration of the measurement procedure. (B) Schematic illustration of polymeric conformational change during compressive loading and unloading procedures.

Fig. 14 shows cyclic tensile testing of cfGel-Hydrogels. Samples (5wt% total polymer concentration, R = 5:5, l = 20 mm, w = 6.5 mm, h = 1 mm) were secured with two mechanical clamps set apart at a distance of ~6.5 mm and stretched at increasingly larger intervals until failure.

Fig. 15 shows (A) Correlation between volume swelling ratio (Q) and storage modulus (G') of cfGel-Hydrogels with 5 wt% fixed total polymer content and varying weight ratio between cfGel-NB and cfGel-SH (R).

Fig. 16 shows (A) Live/Dead staining image of cell-laden printed constructs on day 7. (B) Left: the "Top view" image in Fig. 7B-iii (day 14 immunofluorescence image); middle and right: Zoom-in images of the detailed printed features from left panel.

Fig. 17 shows representative rheological time-sweep measurements of cfGel-Hydrogels with the same content of polymer (5 wt%) but varied weight ratios between cfGel-NB and cfGel-SH polymers (R).

*Description of the Tables*

Table 1 shows the standards that were used in Ellman's reagent assay

Table 2 shows measurement of [-SH] contents at four different cfGel-SH concentrations. Examples

The present invention relates to a two-component hydrogel consisting of norbornene- and thiol-modified cold water fish gelatine. By combining the two components in an approximately 1:1 ratio and subsequent UV-crosslinking in the presence of a photoinitiator (for example, I2959), a hydrogel demonstrating non-swelling properties in physiological media (e.g. PBS, DMEM) is obtained. Other than in most thiol-norbornene photoclick reactions, both norbornene and thiol are chemically linked to their respective gelatine, omitting the need for small crosslinking components with potential cytotoxicity, or other macromolecular crosslinkers derived from other types of polymers that could lead to an inhomogeneous polymer matrix.

The first step, which is the chemical modification of gelatine with norbornene and thiol is a well-established reaction that can be conducted in different ways. The reaction chosen for our invention is described in Figure 1. Following the modification, the material is dialysed over the course of 7 days and subsequently freeze-dried to obtain the final functional gelatine product.

The second step consists of the preparation of the precursor solution. Non-swelling behaviour of the final hydrogel appears to be largely independent of the final concentration of the precursor solution (5wt%, 7.5wt% and 10wt% were tested), with the deciding factor being the ratio between the two components, which should be around 1:1 (likely dependent on the degree of substitution of norbornene- and thiol-groups obtained during the chemical modification).

The third step consists of the UV-crosslinking of the precursor solution. Crosslinking was accomplished through the addition of I2959 (0.05%) and exposing the precursor solution to 365nm UV-light for 2min. Rheological data suggests that gelation already occurs within ~5 seconds post-exposure.

*Example 1: Polymer selection and network design of cfGel-Hydrogels*

Being derived from the primary structural protein of the mammalian extracellular matrix, gelatine retains many of collagen's important characteristics, including the arginine-glycine-aspartate (RGD) sequence, an integrin-binding domain that is instrumental for cell adhesion and for conferring biological functionality to the hydrogel matrix. It has been commonly used in the development of hydrogels for 3D cell encapsulation, bioprinting, and other biomedical applications. While gelatin from mammalian sources represents the most commonly used variant, cold water fish gelatine (cfGel) has recently emerged as a promising alternative owing to its lower gelling and melting temperatures, which allows for dissolution and processing at ambient temperatures, as well as a low immunogenicity and risk of disease transmission in combination with fewer sociocultural concerns regarding its use. Furthermore, due to molecular features such as the lower content of proline and hydroxyproline in cfGel compared to mammalian gelatine, the molecular mobility of polypeptides between hydrogel crosslinking points of cfGel has been found to be higher than that of the latter, representing an important factor for improving cell-matrix remodeling rates, and facilitating stem cell migration in gelatine hydrogels. Some of the reported strategies to facilitate the formation of hydrogels from cfGel involve enzymatic crosslinking in the presence of transglutaminase (TGase), photoinitiated chain polymerization of cold water fish-derived GelMa or thiol-ene "click" crosslinking between norbornene-functionalized cfGel (cfGel-NB) and tetra-thiol polyethylene glycol (4-arm PEG-SH).. However, many of these strategies suffer from drawbacks pertaining to the lack of control over the spatiotemporal gelation of the hydrogels (enzymatic crosslinking), heterogeneous network structures (GelMa) or the involvement of synthetic components and two-component matrices (e.g. 4-arm PEG-SH) that could compromise their potential for biomedical applications. The fully cfGel-based hydrogels (cfGel-Hydrogel) described in this work were developed to overcome such challenges. They were not only able to withstand large cyclic deformations, but also demonstrated notable energy-dissipative capabilities and instantaneous recovery upon mechanical unloading. Such mechanically robust cfGel-Hydrogels were found to offer excellent cytocompatibility. Furthermore, shape retaining cell-laden constructs were fabricated via extrusion-based 3D bioprinting to demon-strate their potential in offering new opportunities for tissue engineering and regenerative medicine approaches (Fig. 1).

*Example 2: Synthesis and rheological characterization of cfGel-Hydrogels*

To develop a fully cfGel-based hydrogel (cfGel-Hydrogel), we synthesized two functional polymers (Fig. 2A), namely norbornene-functionalized cfGel (cfGel-NB) and thiol-functionalized cfGel (cfGel-SH), both of which inherit the intrinsically bioactive characteristics of cfGel while also conveying rapid "click" crosslinking and non-swelling properties unto the resultant hydrogels. The content of functional groups was found to be -170 μmol norbornene [-NB] groups per gram polymer for cfGel-NB (1H NMR, Fig. 8), and 241 pmol thiol [-SH] groups per gram polymer for cfGel-SH (Ellman's reagent assay, Table 2). It is worth mentioning that synthesizing two different gelatine polymers with a singular modification each enables a better control over the final polymeric composition and network structure of the resultant hydrogels compared to the widely used Gel-Ma formulations. This approach also allowed us to avoid commonly used DTT or PEG-based cross-linkers, resulting in "click" hydrogels with biochemically homogeneous network structures (Jasper Van Hoorick et al, Biofabrication, 13 015017, 2021). Upon UV exposure ($\lambda$ = 365 nm, 10 mW/cm2), cfGel-Hydrogels formed rapidly within ~10 seconds (Fig. 2B), demonstrating the excellent crosslinking efficiency of thiol-ene "click" reactions between cfGel-NB and cfGel-SH. The rapid curing of cfGel-Hydrogel was not affected by the mass ratio between cfGel-NB and cfGel-SH, as revealed by oscillatory time-sweep rheological measurements (Fig.9 and Fig. 17). The ratios between cfGel-NB and cfGel-SH (R, by weight) tested include R = 8:2, 7:3, 6:4, 5:5, 4:6, 3:7 and 2:8. For all tested formulations (constant total polymer concentration of 5 wt%) with different R, a sharp increase in G' within the first ~10 s following UV-light exposure was observed, except for R = 8:2, which lacked a sufficient amount of thiol groups to facilitate crosslinking of cfGel polymers into a 3D network and was thus excluded from further analyses (Fig. 17). This R-independent rapid gelation (Fig. 9) represents one of the characteristics of "click" crosslinking and the resulting step-growth polymerization.

By adjusting R, the stiffness of cfGel-Hydrogels could be altered without changing the overall composition (5 wt% cfGel polymers) or affecting the rapid curing feature. A concomitant increase of G' (storage modulus) was observed as the amount of cfGel-SH increased (R from 7:3 to 4:6). Beyond this range (R from 3:7 to 2:8), excessive cfGel-SH led to a reduction in G' (Fig. 2C). This marks a dissimilarity to GelMa hydrogels, which usually require different degrees of chemical functionalization to achieve a difference in stiffness if the polymer concentration is fixed. It is noteworthy that the optimal best R for the highest G' was found to be 4:6. However, based on our quantification of the degree of functionalization for each polymer, the optimal ratio (R) was expected to be around 6:4, which ensures the correct [-NB]:[-SH] stoichiometry (Materials and Methods, Rheological Measurements). This deviation suggests the formation of additional disulfide bonds between excessive thiol moieties, which could result in additional crosslinking between cfGel polymers.

As the "click" crosslinking provides a homogeneous network structure, the excellent structural strength of such cfGel-Hydrogels was demonstrated by the broad linear viscoelastic (LVE) range (Fig. 2D). The formed hydrogels were capable to withstand strains of up to 100% measurements. It was observed that the drop in modulus at high strainlevels was due to adhesive failure between the parallel plate geometry of the rheometer and the hydrogels, rather than due to cohesive failure of the gels themselves. This non-brittle characteristic was demonstrated by all formulations with total cfGel polymer concentrations ranging from 5 wt% to 10 wt% (Fig. 10 and Fig. 11). Furthermore, their viscoelastic behavior under small oscillatory shear strain (r = 1%) featured very low damping, with damping factors, f <<1 (Fig. 2C), suggesting that the homogeneous single-network matrix exhibits good resistance to plastic deformation. Frequency-sweep data further revealed such low-damping behavior until the angular frequency reached 5 s-1, (Fig. 2E, and Fig. 11). At higher frequencies, an increase in G" was observed, indicating insufficient relaxation of the cfGel network under fast shearing.

*Example 3: Non-swelling property of cfGel-Hydrogels under physiological conditions*

As shown in Fig. 3A, cfGel-Hydrogels composed of a fixed initial cfGel polymer content (5 wt%) with R ranging from 7:3 to 2:8 exhibited limited swelling or de-swelling, characterized with swelling ratio (SR) within 50% under physiologically relevant conditions (in PBS, 37°C). We found that formulations with R = 7:3 and R = 6:4 experienced the highest swelling ratio (SR: 42.9 ± 10.1% and 31.5 ± 4.3 %, respectively). While shifting towards cfGel-SH-dominant ratios (R = 4:6, 3:7 and 2:8), hydrogels experienced a concomitant decrease in weight due to de-swelling (SR = -17.3 ± 12.4 %, -17.9 ± 8.9 % and -18.2 ± 4.8 %, respectively). Notably, hydrogels with R = 5:5 exhibited only a negligible weight change after 24 h (SR = -3.2 ± 3.1 %), making them essentially non-swelling. Along with the decrease of R from 7:3 to 4:6, an accompanying decrease of swelling was observed, which is in accordance with the increase in G' of the as-prepared hydrogels (Fig. 2C, and Fig. 15), which implicated increased crosslinking densities. Interestingly, while a further decrease in R caused a reduction in G' due to the insufficient amount of cfGel-NB to mediate effective crosslinking, it did unexpectedly not result in increased swelling. Formulations with comparable G' (R= 6:4 and R = 2:8) displayed different swelling behaviors, implying that although the overall makeup of the hydrogels remained the same (5 wt% cfGel with similar crosslinking density), the difference in residual functional groups ([-NB] or [--SH] groups) in as-prepared hydrogels can influence their swelling behaviors. This suggested that although residual [-SH] groups did not contribute to rapid thiol-ene crosslinking (initial G' of the as-cured cfGel-Hydrogels), they could induce post-curing slow formation of additional crosslinks (e.g. disulfide bonds), thereafter affecting the swelling behavior. It should be further noted that albeit the swelling

ratios of 4:6, 3:7 and 2:8 demonstrate similar values, their respective SD are comparatively high. Regardless of variations, hydrogels prepared from all tested formulations showed a limited capacity for swelling (Fig. 3A). We chose to perform subsequent studies with the formulation R = 5:5, as it demonstrated only a negligible change in weight under physiologically relevant conditions, thus featuring the non-swelling properties desired for retaining hydrogel geometry and mechanics. Therefore, unless stated otherwise, the term "cfGel-Hydrogel" will henceforth refer to hydrogels with R = 5:5. As shown in Fig. 3B, the non-swelling behavior was not affected by the total cfGel polymer concentration. cfGel-Hydrogels prepared with 5 wt%, 7.5 % and 10 wt% polymer precursors showed similar swelling behavior (SR < $\pm$10%) after 168 h of incubation in PBS (Fig. 12). To gain more insight into the underlying mechanism that drives the swelling behavior of cfGel-Hydrogels, we performed a series of swelling studies in PBS solutions of varying concentrations, ranging from 8$\times$ (with 8 times higher salt concentration than purchased PBS) to 0.125$\times$, as well as ultrapure water (UPW) (Fig. 3C). After 24 h, cfGel-Hydrogels exposed to concentrated solutions of PBS exhibited negative swelling (SR = -17.7 $\pm$ 0.4%, -9.0 $\pm$ 3.4% and -12.7 $\pm$ 2.4% for 8$\times$, 4$\times$ and 2$\times$ PBS respectively, Fig. 3D). In contrast, cfGel-Hydrogels showed increased swelling in PBS solutions of lower ionic strength ($\leq$1$\times$), with SR = 1.9 $\pm$ 0.7%, 22.2 $\pm$ 3.0%, 41.7 $\pm$ 12.1%, and 96.4 $\pm$ 3.6% for 1$\times$, 0.5$\times$, 0.25$\times$, and 0.125$\times$ respectively.

In sharp contrast to their swelling behavior in PBS, cfGel-Hydrogels experienced significant degrees of swelling in UPW, reaching SR of 605.5 $\pm$ 11.0 %, around 5-fold higher than what was observed in 0.125x PBS. This indicated that the presence of even a small amount of salts can significantly restrict the swelling of cfGel-Hydrogels. We further found this phenomenon to occur irrespective of pH values. Without adjusting the pH of UPW (5.83), the addition of NaCl (0.14 M) or CaCl2 (0.07 M) demonstrated comparative effectiveness to PBS (pH7.5) in reducing the swelling of cfGel-Hydrogels (Fig. 3E). While we observed similar swelling ratios of cfGel-Hydrogels in NaCl (SR = -15.7 $\pm$ 1.8%) compared to that in PBS, cfGel-Hydrogels exposed to CaCl2 demonstrated a more pronounced de-swelling (SR = -40.4 $\pm$ 2.6%), accompanied by a greater reduction in volume and the adoption of an opaque appearance. This could be due to the interaction between divalent Ca2+ ions and the carboxylic groups of gelatine, which resulted in a reduced number of free carboxylic acid groups available for hydrogen bonding with the surrounding water molecules. These additional interactions can further facilitate polymer chain aggregation within the hydrogels, which leads to the observed decrease in transparency.

It was recently reported that ionic strength could have a significant impact on conformational fluctuations of gelatine polymers in solutions. Herein, we demonstrate that the responsiveness to salts is also present in cfGel polymers crosslinked into a 3D network, which could be explained by the osmotic pressure-mediated flow between the latter's interior and exterior environment. We hypothesize that PBS containing higher salt concentrations (> 1x) elicited negative swelling by extracting water from the interior of the hydrogel, whereas PBS with lower salt concentrations (< 1x) had the opposite effect. To validate this hypothesis, we exposed cfGel-Hydrogels to 1$\times$ PBS, UPW and isotonic (0.25 M) solutions of sucrose (Fig. 3F). Both PBS and 0.25 M sucrose constitute isotonic media, thus neither of them should elicit any osmotic pressure based solely on the difference in solute-concentration between the exterior and interior of the gels. The distinguishing factor in this case is the low ionic strength of the sucrose solution as opposed to PBS. Our data shows that swelling of cfGel-Hydrogels in an isotonic sucrose solution is comparable to that in UPW. This indicates that altering the chemical potential of a solution by in-creasing the concentration of solutes does not affect the swelling behavior of cfGel-Hydrogels as long as there is no concomitant increase in ionic strength. Instead, the swelling behavior of cfGel-Hydrogels appears to be closely tied to polymeric conformational responses of the network to the ionic content of its surrounding medium.

Both gelatine in general and fish gelatine specifically have been reported to experience salt concentration-dependent structural, physicochemical and mechanical alterations when exposed to monovalent or polyvalent salts. Conformational fluctuations are attributed to the changing dynamics between the electrostatic interactions of salt ions and the charged polymer backbone, altering the screening-off of long- and short-range electrostatic repulsions and effectuating tighter or looser association of gelatine $\alpha$-chains. Furthermore, as the concentration of salt increases, competition for water molecules among the ionic and polymeric constituents causes a series of transitions in the hydration state of gelatine, accompanied by both chain shrinkage and expansions. When salt concentrations reach a certain threshold, electrostatic interactions can become strong enough to break and restructure hydrogen bonds and alter the secondary structure of gelatine. Apart from the concentration, the type of salt ion and its location in the Hofmeister series can marginally determine the solubility of gelatine and other proteins in aqueous solutions. The introduction of physical crosslinks through the salting-out effect mediated by kosmotropic ions such as ammonium sulfate has been previously employed to increase the mechanical properties of pre-formed gelatine networks by removing the hydration waters of polymer chains and causing their folding or precipitation. It is noteworthy that we do not observe SR fluctuations of cfGel-Hydrogels with increasing salt concentrations even though changing electrostatic dynamics reportedly facilitate transitions between polymer chain shrinkage and expansion. Furthermore, we observed the most significant degree of de-swelling accompanied by a loss of transparency for cfGel-Hydrogels incubated in $CaCl_2$, despite the chaotropic nature of $Ca^{2+}$ presumably promoting the chain expansion effect. This implies that gelatine conformational responses to salt concentrations behave differently in a network structure compare to those in a solution state.

*Example 4: Robust mechanical properties of cfGel-Hydrogels*

Based on previously established knowledge on the benefits of restricted swelling for the preservation of hydrogel mechanical strength, we expected cfGel-Hydrogels subjected to physiologically relevant conditions to demonstrate superior mechanics by virtue of their non-swelling behavior compared to their swollen counterparts exposed to UPW. As revealed by cyclic compressive tests, cfGel-Hydrogels were able to withstand multiple cycles of high-strain level compressions (80%) in PBS, without incurring cohesive failure or visible signs of damage (Fig. 13). The mechanical robustness under high-strain compressions was further confirmed by the overlap of consecutive loading curves (Fig. 4A). In contrast, cfGel-Hydrogels that experienced swelling as a consequence of prior exposure to UPW were not capable of withstanding the same levels of compressive deformation, but instead suffered cohesive failure and structural breakdown (Fig. 4B).

Our results are in line with previous reports on the detrimental effects of swelling on the mechanical properties of hydrogels and further emphasize on the importance of the non-swelling behavior exhibited by cfGel-Hydrogels in tolerating excessive deformation.

Moreover, cfGel-Hydrogels in PBS consistently exhibited high levels of hysteresis (>40%) between consecutive loading-unloading cycles, thus revealing their capacity for dissipating massive loading energies (Fig. 4A). Importantly, compressive cycles were performed in close succession without any intermediate waiting times. The observation of nearly identical loading curves indicates an instantaneous recovery of the hydrogel network structure upon unloading, which was not observed for cfGel-Hydrogels swollen in UPW (Fig. 4B). The restricted swelling of cfGel-Hydrogels in PBS suggests the existence of unexpanded cfGel polymer chains providing the hydrogel network with a higher apparent crosslinking density ($N \propto 1/Mc$) compared to cfGel-Hydrogels in UPW, where N is the crosslinking density (numbers of elastically effective polymer strands per unit volume), and Mc is the apparent average molecular weight (Mw) of elastically effective polymer strands in between two neighboring crosslinking points. According to the rubber elasticity theory, the Young's modulus (E) of a chemically crosslinked cfGel-Hydrogel should be inversely proportional correlated to Mc ($E \propto 1/Mc$). Experimental measurements confirmed the higher Young's moduli of cfGel-Hydrogels in PBS (EPBS) compared to UPW (EUPW), with EPBS (14.5 ± 1.3 kPa) being around three times higher than EUPW (4.4 ± 0.3 kPa) (Fig. 4C).

Depending on their intended application, hydrogels might not experience a complete relaxation during consecutive instances of mechanical stress. Taking this factor into consideration, it is important for such hydrogels to be able to resist mechanical deterioration during cyclic deformations where residual strains might remain. Subjecting cfGel-Hydrogels in PBS to cyclic compressions with various residual strains (Fig. 4D, insert) yielded identical peak forces for subsequent cycles as observed for the first loading curve (at peak strain $\varepsilon p = 80\%$). Additionally, consecutive loading-unloading curves were found unaltered under all residual strains ($\varepsilon r = 20 - 70\%$) (Fig. 4D). Conversely, the degree of hysteresis was dependent on the strain imposed on the hydrogels during cyclic deformation ($\Delta\varepsilon = \varepsilon p - \varepsilon r$), showing more pronounced hysteresis with a concomitant increase of $\Delta\varepsilon$. For $\varepsilon r = 70\%$ ($\Delta\varepsilon = 10\%$), only minor hysteresis was observed. Lowering residual strains down to 60% ($\Delta\varepsilon = 20\%$) already resulted in a considerably higher hysteresis, indicating substantial energy dissipation and recovery. The ability of cfGel-Hydrogels to withstand extended periods of non-resting compressive stress without suffering debilitating mechanical performances constitutes an essential attribute for their potential use in biomedical applications involving excessive dynamic loadings. In addition, cfGel-Hydrogels can also withstand uniaxial stretching cycles, showing no signs of fatigue during repeated cycles of stretching with tensile strains up to 168% (Fig. 14).

The effect of restricted swelling on the mechanical properties of cfGel-Hydrogels can be explained by the conformational change of cfGel polymer chains. Upon compressive loading in PBS, forced chain expansions dissipate the loading energy and prevent the rupture of the polymer network (Fig. 13B) . The conformational retraction of the polymer chains upon unloading rapidly restores the original state of the 3D cfGel polymer network structure, thus giving rise to both the energy-dissipating and fast-recovering properties we observed for cfGel-Hydrogels in PBS (Fig. 5). To reveal the conformational shrinkage of cfGel polymers in PBS compared to those in UPW, we defined the averaged distance between two neighboring crosslinking points as d. According to the rubber elasticity theory, the relative change of cfGel-Hydrogel Young's modulus in different swelling media can be expressed as:

$$\frac{E_{UPW}}{E_{PBS}} = \frac{M_{C-PBS}}{M_{C-UPW}}$$

where $M_{c\text{-}PBS}$ and $M_{c\text{-}UPW}$ are the apparent average $M_w$ of polymer strands in between two crosslinking points in PBS and UPW respectively. As *d* can be correlated to $M_c$ as ($d \propto M_c^{0.57}$), it can be estimated that:

$$\frac{d_{PBS}}{d_{UPW}} = \left(\frac{M_{C-PBS}}{M_{C-UPW}}\right)^{0.57} = \left(\frac{E_{UPW}}{E_{PBS}}\right)^{0.57} = \left(\frac{4.4\ kPa}{14.5\ kPa}\right)^{0.57} = 0.51$$

where $d_{UPW}$ and $d_{PBS}$ are the distances between two neighboring crosslinking points in UPW and PBS respectively. This reflected the substantial conformational shrinkage (49%) of cfGel polymer chains in PBS compared to those in UPW, (Fig. 5), which is key to the abovementioned energy dissipating and rapid recovery properties of cfGel-Hydrogel in PBS.

Moreover, the equilibrated volume of cfGel-Hydrogels in UPW compared to that in PBS can be estimated as below:

$$\frac{V_{UPW}}{V_{PBS}} = \left(\frac{d_{UPW}}{d_{PBS}}\right)^3 = \left(\frac{1}{0.51}\right)^3 = 754\%$$

where $V_{UPW}$ and $V_{PBS}$ is the equilibrated volume of cfGel-Hydrogels in UPW and PBS respectively. This is close to our observed swelling behavior **(Fig. 3D)**, where $W_{PBS} = 1.02 \times W_0$, and $W_{UPW} = 7.06 \times W_0$, thus giving rise to the observed value of $W_{UPW}/W_{PBS}$ = 692%. Considering the high water content of the hydrogels (initially 95 wt%), their overall density should be close to that of water, therefore $V_{UPW}/V_{PBS}$ can be estimated as the measured $W_{UPW}/W_{PBS}$.

*Example 5: 3D encapsulation and bioprinting of human dermal fibroblasts*

Given the bioactive properties of a fully gelatin-based matrix in tandem with the mild reaction conditions inherent to the thiol-ene click reaction, 3D encapsulation and culturing of living cells with cfGel-Hydrogels was realized. This distinguishes cfGel-Hydrogels from existing non-swelling hydrogels made from fully synthetic polymers, or those fabricated via multi-step procedures. Primary human dermal fibroblasts (HDFs) were encapsulated in cfGel-Hydrogels by UV-curing of cell-suspended precursor solutions with cfGel polymer concentrations of 5 wt%, 7.5 wt% or 10 wt% (Fig. 6A). Metabolic activity of encapsulated HDFs showed only minor differences within the first three days of culture between hydrogels prepared from either cfGel polymer concentration, indicating an initially limited cell proliferation (Fig. 6B). After seven days, HDFs demonstrated significantly elevated levels of metabolic activity in all hydrogels when compared to day three, as well as significantly higher values with decreasing polymer concentrations. These observations could be substantiated by a live/dead assay and immunofluorescence staining for the actin cytoskeleton, which revealed a high cell viability and more pronounced spreading behavior of HDFs in 5 wt% and 7.5 wt% cfGel-Hydrogels compared to 10 wt% (Fig. 6C). We attribute this to the more restrictive environment imposed by the stiffer network structure of 10 wt% gels, which offers increased resistance to cell spreading and proliferation. That aside, the majority of cells were found viable in all examined cfGel-Hydrogels for the entire cultivation period, thus emphasizing the cytocompatible nature of both the material components and the fabrication method.

The abovementioned collective properties of cfGel-Hydrogels make them a promising material for 3D bioprinting, especially with regard to cell culturing within shape-retaining 3D constructs. In order to evaluate the printability, shape fidelity and shape-retaining properties of cfGel-Hydrogels, we used extrusion-based bioprinting and measured the degree of resemblance between the original computer-assisted design (CAD) and the printed structure, as well as the viability of included cells during post-printing long-term culturing. Based on the results of the 3D cell encapsulation, we chose 5 wt% cfGel as the polymer concentration for our printing studies. Due to the low viscosity of the precursor solutions, we employed freeform reversible embedding of suspended hydrogels (FRESH) for 3D printing, whereby bioinks are directly deposited into viscous support baths. We opted for the use of an agarose support bath formulation in accordance with previous descriptions on account of its non-toxic nature and ease of post-printing removal. For our target structure we chose a simple multi-layered grid (9 mm length $\times$ 9 mm width $\times$ 0.6 mm thickness), as it would allow us to easily detect dimensional alterations to the individual filaments over time. The printed grids displayed no visually significant structural alterations or noticeable signs of degradation when compared to their as-printed morphology during incubation under physiologically relevant conditions for up to two weeks (Fig. 7A). Measuring and comparing the area of imaged grids using QuPath image analysis software revealed the shape-retaining property of the 3D printed construct (Fig. 7C) (Schwab, A., et al., Chem Rev, 120. 19 (2020), 11028).

We subsequently evaluated the effects of the printing of cfGel-Hydrogels on the viability of incorporated HDFs. While equipped with the same features as their cell-free counterparts, dimensions of cell-containing grids were altered to have an increased thickness and a smaller circumference (6 mm length $\times$ 6 mm width $\times$ 1 mm thickness), in order to assess printing resolution for smaller-scale constructs. Live/dead imaging of cells after both one day and one week post-printing culturing showed no detrimental influence of the printing process on cell viability (Fig. 7Bi, and Fig. 16A). This aspect likely benefitted from the low viscosity of the cfGel polymer precursor solutions, which reduced shear forces exerted onto cells during extrusion. Moreover, we did not observe major alterations to the shape of the printed structures in the

presence of HDFs after one week of culturing (Fig. 7B-ii). However, we did observe some degree of curving or bending of the printed grids by day 14, which might be due to the contractile forces exerted by the incorporated fibroblasts (Fig. 7B-iii). Staining for actin/neclei and vimentin revealed 3D spreading and homogeneous distribution of collagen I-depositing HDFs throughout the printed structures. While the contractile forces exerted by encapsulated cells could be visualized by curving or bending of the cell-laden grids, structural integrity and overall shape of the 3D bioprinted constructs, including more detailed features such as individual filaments and gaps, were maintained even after 14 days of culture (Fig. 16B). Therefore, as an intrinsically non-swelling material platform, cfGel-Hydrogels offer new opportunities for investigating long-term cell-matrix interactions with minimum interference from swelling.

*Example 7: Discussion*

To overcome the current challenges faced by mechanically robust and shape-retaining cytocompatible hydrogels in the field of 3D bioprinting, we developed a fully cold-water fish gelatin-based "click" hydrogel (cfGel-Hydrogel) composed of a single-network structure that can be formed via simple one-step gelation while also demonstrating significant resistance to swelling under physiologically relevant conditions. The non-swelling characteristic, resulting from salt-induced conformational shrinkage of the gelatin polymer, imparts energy dissipating and rapid recovery properties to cfGel-Hydrogels. The excellent cytocompatibility allows for their application in 3D cell encapsulation and long-term culturing, as well as 3D printing of living cells. The resistance of the printed cell-laden structures to media-related swelling resulted in the preservation of the initial scaffold design, thus making cfGel-Hydrogels a promising platform for precise engineering of 3D artificial tissues and for investigating the impact of geometric cues on cell behavior and disease modelling.

*Example 8: Materials and Methods*

*Materials*

Materials used in this study include Gelatin from cold water fish skin (Sigma-Aldrich, G7041-500G, Lot. SLCH9658), Phosphate buffered saline (PBS, Sigma-Aldrich, P4417-100TAB), 2-Hydroxy-4'-(2-hydroxyethoxy)-2-methylpropiophenone (Irgacure 2959, Sigma-Aldrich, 410896-10G), Dulbecco's Modified Eagle's Medium (DMEM, Sigma-Aldrich, D5796), Dulbecco's Phosphate Buffered Saline (DPBS, Sigma-Aldrich, D8537-500ML), fetal bovine serum (Sigma-Aldrich, F9665, Lot. 0001655440), penicillin-streptomycin (Sigma-Aldrich, P4458-100ML, Lot. 0000192944), L-glutamine (Sigma-Aldrich, G7513-100ML, Lot. RNBM0086), agarose (VWR, 733-1537), lithium phenyl-2,4,6-trimethyl-benzoylphosphinate (LAP, Sigma-Aldrich, 900889-1G), rhodamine B isothiocyanate (Sigma-Aldrich, R1755-100MG), 10% neutral buffered formalin (Sigma-Aldrich, HT501128), calcein AM (Sigma-Aldrich, 206700-1MG), ethidium homodimer (Sigma-Aldrich, 46043-1MG-F), Cysteine Hydrochloride Monohydrate (Thermo Scientific, Product No. 44889), Ethylenediaminetetraacetic acid disodium salt dihydrate (Sigma-Aldrich, E5134), Bovine Serum Albumin (Sigma-Aldrich, A9647-50G), Cis-5-norbornene-endo-2,3-dicarboxylic anhydride (19.3 g, Sigma-Aldrich, 247634-5G).

*Synthesis of functional gelatine polymers*

- *Norbornene-functionalized cold water fish gelatin (cfGel-NB):*

Cold water fish gelatin (10 g) was dissolved in 100 mL Phosphate buffered saline at 50 °C under constant stirring. Cis-5-norbornene-endo-2,3-dicarboxylic anhydride (19.3 g) was added to the gelatin solution and the pH value of the mixture was adjusted to between 7.5-8.0 by adding NaOH (aq. 1 M) until the solid cis-5-norbornene-endo-2,3-dicarboxylic anhydride was fully dissolved. The reaction was continued for 48 hours with pH value maintained between 7.5-8.0. Subsequently, the reaction was cooled down to room temperature (23 °C), and dialysed against DI water for 1 day and nanopure water for 2 days (Mw. cutoff 6-8kD). A foam-like product (8.5 g) was obtained after freeze-drying, and analysed by proton nuclear magnetic resonance (1H NMR, Fig. 8).

- *Thiolated cold water fish gelatin (cfGel-SH):*

Cold water fish gelatin (20 g) was dissolved in 0.1 M sodium bicarbonate buffer (pH 10, 200 mL) at room temperature under constant stirring. Ethylenediaminetetraacetic acid (EDTA, 87.6 mg) was added to the solution and the mixture was purged with argon gas for 2 minutes. N-acetylhomocystein thiolactone (6.13 g) was added to the solution under argon gas protection. The reaction was continued at room temperature for 12 hours. Afterwards, undissolved solids were removed by filtration. The solution was concentrated to 150 mL by rotary evaporation and subsequently freeze-dried, yielding a foam-like product. Analysis was conducted using the Ellman's reagent assay (Thermo Fisher Scientific).

*Ellman's reagent assay*

To evaluate the degree of functionalization of cfGel-SH, we performed Ellman's reagent assay to quantify the number of sulfhydryl groups within the polymer using cysteine standards. Cysteine standards were prepared by dissolving varying amounts of Cysteine Hydrochloride Monohydrate in Reaction Buffer (0.1 M sodium phosphate, pH 8.0, containing 1 mM Ethylenediaminetetraacetic acid disodium salt dihydrate. The standards in Table 1 were used.

Next, a set of test tubes, each containing 10 μL of Ellman's Reagent Solution and 0.5 mL of Reaction Buffer were prepared, followed by the addition of 50 μL of each standard or cfGel-SH solution (0.5 mg $mL^{-1}$, 1 mg $mL^{-1}$, 10 mg $mL^{-1}$, 100 mg $mL^{-1}$ in PBS). After mixing, samples were incubated at room temperature for 15 min before being added in triplicates (100 μL each) into the wells of a 96-well plate (Product No. 92096) and absorbance at 405 nm was measured using a $Mithras^2$ LB 943 Multimode Microplate Reader. Values obtained for cysteine standards were plotted to create a standard curve, from which the experimental sample concentrations were determined (Table 2).

*Hydrogel molding with different formulations*

Hydrogel precursor solutions were prepared by dissolving lyophilized cfGel-NB and cfGel-SH in 1×PBS at room temperature through alternating use of vortexing and centrifugation until a clear homogeneous solution was obtained, after which 2-Hydroxy-4'-(2-hydroxyethoxy)-2-methylpropiophenone (Irgacure 2959, stock solution 0.5% (w/v) in UPW) was added to yield the hydrogel precursor solutions with final Irgacure 2959 concentration at 0.05 % (w/v), cfGel-NB and cfGel-SH concentrations as defined for different formulations. For example, for hydrogels containing 5 wt% of polymers at a cfGel-NB to cfGel-SH weight ration of 5:5, the precursor solution would contain 12.5 mg cfGel-NB and 12.5 mg cfGel-SH in 425 μL PBS with 50 μL Irgacure 2959 stock solution.

To form the hydrogels, 40 μL of the solution was cast into a custom-made mold consisting of a rectangular sheet of PDMS of 1 mm thickness containing holes of 6 mm diameter pressed onto a glass slide. To allow for easier removal of crosslinked hydrogels, the glass slide was treated with Sigmacote® (Sigma-Aldrich, SL2). In cases where a flat hydrogel surface was required, the loaded mold was covered with a second PDMS sheet prior UV-irradiation for 2 min at room temperature using a 12W (VL-206.BL, 2×6W, 365 nm) UV light source spaced -3.5-0 cm away from the mold to ensure the intensity of 7.5-10 $mW/cm^2$ throughout the whole mold. Adding 1×PBS between the two PDMS-sheets to lubricate the hydrogels allowed for easier removal of the components of the mold without incurring damage to the gels. We found that using a cell-scraper was the easiest way to remove cfGel-Hydrogels from the glass surface.

The weight ratio between cfGel-NB and cfGel-SH for correct [-NB]:[-SH] stoichiometry is defined as Rs. A formulation with Rs ensures the equal molar concentration between [-NB] and [-SH] groups, thus represents the theoretically optimum formulation for the highest hydrogel modulus under fixed polymer concentration. Since the [-NB] content and [-SH] content of each gram functional polymer is known to be 170 μg and 240 μg, Rs can be calculated as 240/170 (Rs~6:4).

*Rheological measurements*

Rheological measurements were conducted using an Anton Paar Physica MCR 301 Rheometer equipped with a PP7 parallel plate geometry (diameter 7 mm, Cat No. 10636, without Toolmaster) and a UV light curing system (P-PTD200/GL) in air at 25°C. Oscillatory time-sweep measurements were performed for 4 min at a frequency of 1 Hz and were prepared by adding 60 μL of precursor solution onto the UV light curing stage and lowering the geometry to 1 mm, resulting in the precursor solution filling out the entire space between PP7 plate and the sample stage. Once in place, the hood of the Peltier temperature control system was lowered over the sample and the measurement was started. After 30 seconds of measuring, the precursor solution was exposed to 365nm UV-irradiation (10 mW) using an OmniCure UV curing system (Exfo, Model No. S1000-IB). Irradiation was stopped after 2.5 min and the measurement continued for another 1 min before termination.

Frequency sweep measurements were performed at a frequency of 10-0.01 Hz directly following time-sweep measurements. Strain sweep measurements were conducted after frequency sweep measurements at a frequency of 1 Hz and an increase in strain from 1-1000% until a clear drop in modulus was detected.

*Swelling ratio measurements*

Swelling ratio (SR) was evaluated by determining the change in weight of as-prepared "relaxed" and swollen hydrogels at specific timed intervals. Following separation from the mould, any excess liquid was removed from the hydrogels before their initial weight $W_0$ was recorded. Hydrogels were subsequently placed in 12-well TPP tissue culture test plates (TPP, Product No. 92012) and incubated in either variably concentrated PBS or UPW at 37°C for up to 24 h. The weight of the swollen hydrogels $W_t$ was recorded after 1 h, 2 h, 4 h, 6 h and 24 h by removing them from the respective

incubation medium and drying off any excess liquid. The swelling ratio was calculated by applying the following formula:

$$Swelling\ ratio\ (SR)\ (\%) = \frac{W_t - W_0}{W_0} \times 100\%$$

*Compression measurements*

Disc-shaped hydrogels (6 mm diameter × 1 mm thickness) were exposed to uniaxial compression using an Anton Paar Physica MCR 301 Rheometer in either PBS or UPW at 37°C. Prior to testing, samples were incubated for 1 h in PBS or UPW at 37°C in a laboratory warming cabinet (Labocult, Model H7 70) to prevent excessive swelling during the measurements, which is especially relevant for UPW-exposed hydrogels. Further swelling after 1 h was prevented by removing most of the incubation medium, leaving only enough to maintain a humid environment to counteract dehydration as samples were kept at 37°C until being measured. Samples were incubated and kept in 12-well tissue culture test plates (TPP, Product No. 92012). For compressive measurements, samples were first transferred into a flat-bottomed plastic petri dish and the diameter was recorded using an Aerospace 150 mm Digital Vernier Caliper. Next, the petri dish was placed on the lower plate of the rheometer while the upper plate slowly (10 μm/s) approached the sample until contact was established, indicated by an increase in the detected normal force. The initial measurement position was then set to be slightly (one or two measurement points) above the first point of positive force detection to avoid excessive preliminary compression. Following contact establishment, the petri dish was filled with either PBS or UPW until the PP25 Parallel-plate (diameter 25 mm, Cat No. 79044) was completely submerged. Both incubation media as well as the PP25 plate were heated to 37°C to prevent temperature fluctuations during the measurements.

Samples were allowed to equilibrate for 5 min in the incubation medium before compressive tests were started. Compression and relaxation were performed at a constant speed of 50 μm/s and frequency of 0.001 Hz for various amounts of time corresponding to 80% compressive strain. The amount of time t required was determined using the following formula:

$$t = \frac{(h_i - h_d) \times 0.8}{0.05},$$

where $h_i$ is the initial height used as the measurement position and $h_d$ the height of the petri dish to be subtracted to obtain the original height of the hydrogel sample $h_s$. Multiplying the original height of the sample by 0.8 reveals the required distance the geometry has to descend in order to achieve 80% compression. By further dividing the distance by the travel speed of 0.05 mm/s, we obtain the amount of time needed per interval of compression or unloading. For measurements, four consecutive cycles of compression were executed without intermediate waiting times between cycles. Hysteresis loss was calculated by integrating the area between loading and unloading curves using Origin.

To determine the Young's modulus of PBS- and UPW-swollen cfGel-Hydrogels, stress $\sigma$ and strain $\varepsilon$ were calculated using the following formulas:

$$\sigma = \frac{F_t \times (h_t - h_d)}{\pi r^2 \times h_s},$$

$$\varepsilon = \frac{h_i - h_t}{h_s} \times 100\%,$$

where $F_t$ and $h_t$ represent the measured force and height (gap size) at any given time during the measurement and $r$ the original radius of the sample.

For residual strain compression, measurement procedures were slightly altered. Instead of defining the interval time for loading and unloading needed based on the travel speed of the PP25 plate, we defined the upper and lower limits of gap size corresponding to the respective percentage of compression, with loading and unloading sequences being separated into different measurement intervals. As soon as the lower limit of the gap size, which corresponded to 80% compression, was reached, the unloading interval would start and lift the geometry to the upper gap size limit, which corresponded to the respective residual strain.

*Tensile mechanical measurements*

Tensile testing was performed using a BioTester 3000 Biaxial Test System (CellScale) equipped with a 0.5 N load cell and a heatable fluid chamber. Rectangular samples with 20 mm length × 6.5 mm width × 1 mm thickness were secured with two mechanical clamps set apart at a distance of ~6.5 mm and lowered into the pre-heated fluid bath (DPBS). Samples were incubated in PBS at 37°C for at least 1 h prior to mounting and afterwards kept at room temperature until used. To prevent damage to the samples during mounting, hydrogels were placed on a supportive structure consisting of a piece of paper until submerged. Afterwards, parts of the supportive structure were removed so as to not interfere with the measurement and samples were given another ~5 min of equilibration time in the heated fluid bath (37 °C) before stretching. Samples were stretched for 5 cycles at a speed of 1 mm/s at increasing intervals of 1 mm (i.e. 5 cycles of 1 mm stretch - 5 cycles of 2 mm stretch - 5 cycles of 3 mm stretch...) until failure. For the first cycle of each interval, a preload of 1.5 mN was applied before stretching. After each interval, the distance was reset to the original position prior to pre-loading.

*Cell culture*

Primary human dermal fibroblasts (HDFs, CELLnTEC, Lot. EB1104281, pooled juvenile donors) were cultured in Dulbecco's Modified Eagle's Medium (DMEM, Sigma-Aldrich, D5796) supplemented with 10% (v/v) fetal bovine serum (Sigma-Aldrich, F9665, Lot. 0001655440), 1% penicillin-streptomycin (Sigma-Aldrich, P4458-100ML, Lot. 0000192944), and 1% L-glutamine (Sigma-Aldrich, G7513-100ML, Lot. RNBM0086) in a humidified environment at 5% $CO_2$ and 37°C. HDFs were used up to passage 10. Cell encapsulations for cytocompatibility evaluation were done with passages 4, 6, and 7. Bioprinting experiments were conducted with passages 4, 5, 6, and 9. Cells were cultured in standard tissue culture plastic flasks (TPP, Product No. 90076) with media changes being conducted twice a week until 80%-100% confluency. Following trypsinization, cells were collected in cell culture medium, pelleted and reconstituted in cfGel-Hydrogel precursor solution at a density of 2'000'000 cells/mL for both cell encapsulation and bioink preparation purposes. Cell-laden hydrogels were molded in the same way as described in section "2.3 Hydrogel molding with different formulations", with the abovementioned amount of cells suspended in the precursor solutions.

*Three dimensional (3D) bioprinting*

Agarose suspension baths were prepared in accordance to previously published descriptions (Prendergast, M. E, et al., Adv Healthc Mater., e2101679, (2021)). Specifically, agarose (VWR, 733-1537) was added to UPW at a concentration of 0.5% (w/w) and autoclaved at 120°C. Directly following autoclaving, the still hot agarose solution was placed on a stir plate and sheared at 700 rpm until it cooled to room temperature and was subsequently stored at 4°C until further use. To prepare suspension baths, the 0.5% (w/w) agarose solution was diluted with sterile Dulbecco's Phosphate Buffered Saline (DPBS, Sigma-Aldrich, D8537-500ML) to a concentration of 0.25% (w/w) and centrifuged at 500 rpm for 5 min before being added to 12-well plates.

Both cell-laden and cell-free constructs were printed using a Bio X6 extrusion printer (CELLINK). Inks used for cell-free printing consisted of 2.5% (w/w) cfGel-NB and 2.5% (w/w) cfGel-SH with 10% (w/w) lithium phenyl-2,4,6-trimethyl-benzoylphosphinate (LAP, 0.5% (w/w), Sigma-Aldrich, 900889-1G) as photoinitiator, 10% (w/w) rhodamine B isothiocyanate stock solution (1% (w/w), Sigma-Aldrich, R1755-100MG) for visualization, and DPBS as the solvent. Cell-free constructs with 9 mm length × 9 mm width × 0.6 mm thickness were printed in a rectilinear pattern with 8% infill at a speed of 10 mm/s and an extrusion rate of 1.5 μL/s using a 27 gauge needle. For each independent repeat, four constructs were successively printed and crosslinked at the same time using an external UV-source (VILBER, VL-206.BL, 2×6W, 365 nm). After printing, constructs were imaged using a confocal microscopy system (Zeiss LSM780) while still encased in the support bath to record the as-printed dimensions. Afterwards, the support bath was removed and the samples were washed 3× with and subsequently submerged in DPBS before another round of image acquisition. Samples were then incubated at 37°C for two weeks in a laboratory warming cabinet (Labocult, Model H7 70) without $CO_2$ control and imaged at day 7 and 14.

For bioprinting, bioinks were prepared by dissolving 2.5% (w/w) cfGel-NB and 2.5% (w/w) cfGel-SH together with 10% (w/w) LAP (0.5% (w/w)) and HDFs (2'000'000 cells/ml) in DPBS. Cell-laden constructs with 6 mm length × 6 mm width × 1 mm thickness were printed in a grid pattern with 24% infill at a speed of 10 mm/s and an extrusion rate of 1.5 μL/s using a 27 gauge needle. For each independent repeat, four constructs were successively printed and crosslinked at the same time using an external UV-source (VILBER, VL-206.BL, 2×6W, 365 nm). Immediately after printing, support baths were removed and samples were washed 3× with sterile DPBS before being submerged in DMEM cell culture medium supplemented with 10% (v/v) FBS, 1% (v/v) penicillin/streptomycin and 1% (v/v) L-glutamine and incubated at 37°C and 5% $CO_2$. Images were taken at day 1 and 7 for live/dead staining, and day 7 and 14 for immunostainings respectively.

*Immunofluorescence staining and analysis*

Immunofluorescence analysis was conducted by fixating cfGel-Hydrogels containing encapsulated HDFs in 10% neutral buffered formalin (Sigma-Aldrich, HT501128) for ~30 min followed by permeabilization treatment with 0.1% Triton X-100 (Sigma-Aldrich, T8787-100ML) for -15 min. Samples were subsequently washed with DPBS 3× for 1 min before being either stored at 4°C or directly processed further. Blocking was performed in 3% BSA (in PBSbovine serum albumin (BSA in DPBS, Sigma-Aldrich, A9647-50G) for at least 1h (ranging up to ~5h) at room temperature or o/n at 4°C before samples were incu-bated in primary antibody solution o/n at 4°C. After secondary antibody incubation for 1-1.5h at room temperature in the dark, samples were washed 3× with DPBS for 1 min. All antibodies were diluted by a factor of 1:200 in DPBS containing 1% fetal bovine serum, with DAPI being diluted by 1:1000. For live/dead staining, samples were incubated with cell culture medium containing 0.1% (v/v) calcein AM (stock: 1mM in DMSO, Sigma-Aldrich, 206700-1MG) and 0.1% (v/v) ethidium bromidehomodimer (stock: 1mM in DMSO, Sigma-Aldrich, 46043-1MG-F) for ~1h at 37°C followed by washing in DPBS 3× for 1 min. Samples were imaged in DPBS *NMR Characterization*

The content of norbornene [-NB] groups was calculated by comparing the integration of the proton signals between unsaturated double bonds ($A_{NB}$) and a DMSO external reference ($A_{DMSO}$). With $M_1$ mg cfGel-NB and $M_2$ mg DMSO dissolved in the sample (DMSO-d6 solution), the NB content ($C_{NB}$, mol) can be calculated using the following formula:

$$\frac{A_{NB}}{A_{DMSO}} = \frac{(2 \text{ x } C_{NB})}{6 \times \frac{M_2}{M_{DMSO}}}$$

where, according to the spectrum,

$$\frac{A_{NB}}{A_{DMSO}} = \frac{1}{113.35}$$

the molecular weight of DMSO is $M_{DMSO}$ = 78.13 g/mol, and $M_1$ and $M_2$ are known from the experiment (50mg and 5 mg respectively). Therefore, the content of norbornene per gram polymer is:

$$\frac{C_{NB}}{M_1} = 170\ \mu\text{mol}$$

*Cited references:*

Muir, V. G., and Burdick, J. A., et al., Biopolymers for the Formation of Biomedical Hydrogels, Chem Rev, 121 (18), 10908-10949, 2021

Yoon, H. J., et al., Cold Water Fish Gelatine Methacryloyl Hydrogel for Tissue Engineering Application, PLoS One, 11 (10), e0163902, 2016

Bellis, S. L., et al., Advantages of RGD peptides for directing cell association with biomaterials, Biomaterials, 32 (18), 4205

Le Thi P, et al., Horseradish peroxidase-catalyzed hydrogelation of fish gelatine with tunable mechanical properties and biocompatibility, Journal of Biomaterials Applications, 34(9), 1216-1226, . 2020

Nitsuwat, S. et al., Fish gelatin as an alternative to mammalian gelatin for food industry: A meta-analysis, LWT, 2021, Vol. 141, 110899

Zhan, Y., et al., Advances in versatile anti-swelling polymer hydrogels, Mater Sci Eng C Mater Biol Appl, 127, 112208, August 2021

CN112220963B

WO2021180795A1

WO2024113021A1

Jasper Van Hoorick et al. , Thiol-norbornene gelatin hydrogels: influence of thiolated crosslinker on network properties and high definition 3D printing, Biofabrication, 13 015017, 2021

Schwab A, et al., Printability and Shape Fidelity of Bioinks in 3D Bioprinting, Chem Rev, 120(19), 11028-11055, 2020

Lee, A., et al., 3D bioprinting of collagen to rebuild components of the human heart, Science, 365 (6452), 482-487,

Prendergast ME, Computational Modeling and Experimental Characterization of Extrusion Printing into Suspension Baths. Adv Healthc Mater. 2022 Apr;11(7):e2101679.

All scientific publications and patent documents cited in the present specification are incorporated by reference herein.

Table 1

| | Volume of Reaction Buffer | Amount of Cysteine | Final Concentration |
|---|---|---|---|
| **Stock Solution** | 2 mL | 5.307 mg | 15 mM |
| **A** | 1.8 mL | 0.2 ml of Stock Solution | 1.5 mM |
| **B** | 0.1 mL | 0.5 mL of A | 1.25 mM |
| **C** | 0.2 mL | 0.4 mL of A | 1.0 mM |
| **D** | 0.3 mL | 0.3 mL of A | 0.75 mM |
| **E** | 0.4 mL | 0.2 mL of A | 0.5 mM |
| **F** | 0.5 mL | 0.1 mL of A | 0.25 mM |
| **G** | 0.1 mL | | Blank |

Table 2

| cfGel-SH (mg/mL) | [-SH] (mM) | [-SH] content (µmol/g polymer) |
|---|---|---|
| **0.5** | 0.12 | 245.317 |
| **1** | 0.24 | 241.006 |
| **10** | 1.23 | 123.246 |
| **100** | 2.59 | 25.876 |

## Claims

1. An aqueous composition comprising:

   a. thiolated gelatine and
   b. norbornene-modified or vinyl-ester modified gelatine

   wherein the gelatine is **characterized by** a gel point of ≤20°C.

2. The aqueous composition according to claim 1, wherein the thiolated gelatine is **characterized by**

   a. between 50 and 500 pmol thiol groups per gram of gelatine, and
   b. between 50 and 500 pmol norbornene or vinyl-ester groups per gram of gelatine.

3. The aqueous composition according to any one of the preceding claims, wherein the number of proline residues in the gelatin is not more than 20% of the total number of amino acid residues in the gelatin.

4. The aqueous composition according to any one of the preceding claims, wherein the total concentration of gelatine

ranges from 3 to 20 % (w/w).

**5.** The aqueous composition according to any one of the preceding claims, wherein the ratio of thiolated gelatine to norbornene-modified gelatine ranges from 1:3 to 4:1.

**6.** The aqueous composition according to any one of the preceding claims, wherein the ratio of thiol groups to norbornene groups in the composition ranges from 2:3 to 3:2.

**7.** The aqueous composition according to any one of the preceding claims, wherein the composition consists of thiolated gelatine and norbornene-modified gelatine and an aqueous solution of electrolytes, **characterized by** an osmolality of: 310 - 350 mOsm/Kg $H_2O$.

**8.** The aqueous composition according to any one of the preceding claims, wherein the gelatine is cold water fish gelatine.

**9.** The aqueous composition according to any one of the preceding claims, further comprising a photoinitiator.

**10.** The aqueous composition according to any one of the preceding claims, further comprising live mammalian cells.

**11.** The aqueous composition according to claim 10, wherein the mammalian cells are selected from the group consisting of skin fibroblasts, induced pluripotent stem cells (iPSC); macrophages, T cells; endothelial cells (EC).

**12.** A method to generate a three-dimensional hydrogel object, particularly an in vitro model for disease modelling/drug testing or an implant, comprising the steps of:

a. providing a composition according to any one of the preceding claims 1 to 11;
b. forming the hydrogel object by bioprinting (extrusion-based or digital light-based (DLP)) or molding/casting to provide the three-dimensional hydrogel object.

**13.** The method according to claim 12, wherein, subsequent to step b, the hydrogel object is incubated in pure water or an isotonic sugar solution, then contacted with a membrane and placed in salt-containing medium.

**14.** A hydrogel implant obtained by a method according to claim 12 or 13.

**15.** A kit comprising dried thiolated cold-water species gelatine and norbornene-modified cold-water species gelatine.

Fig. 1

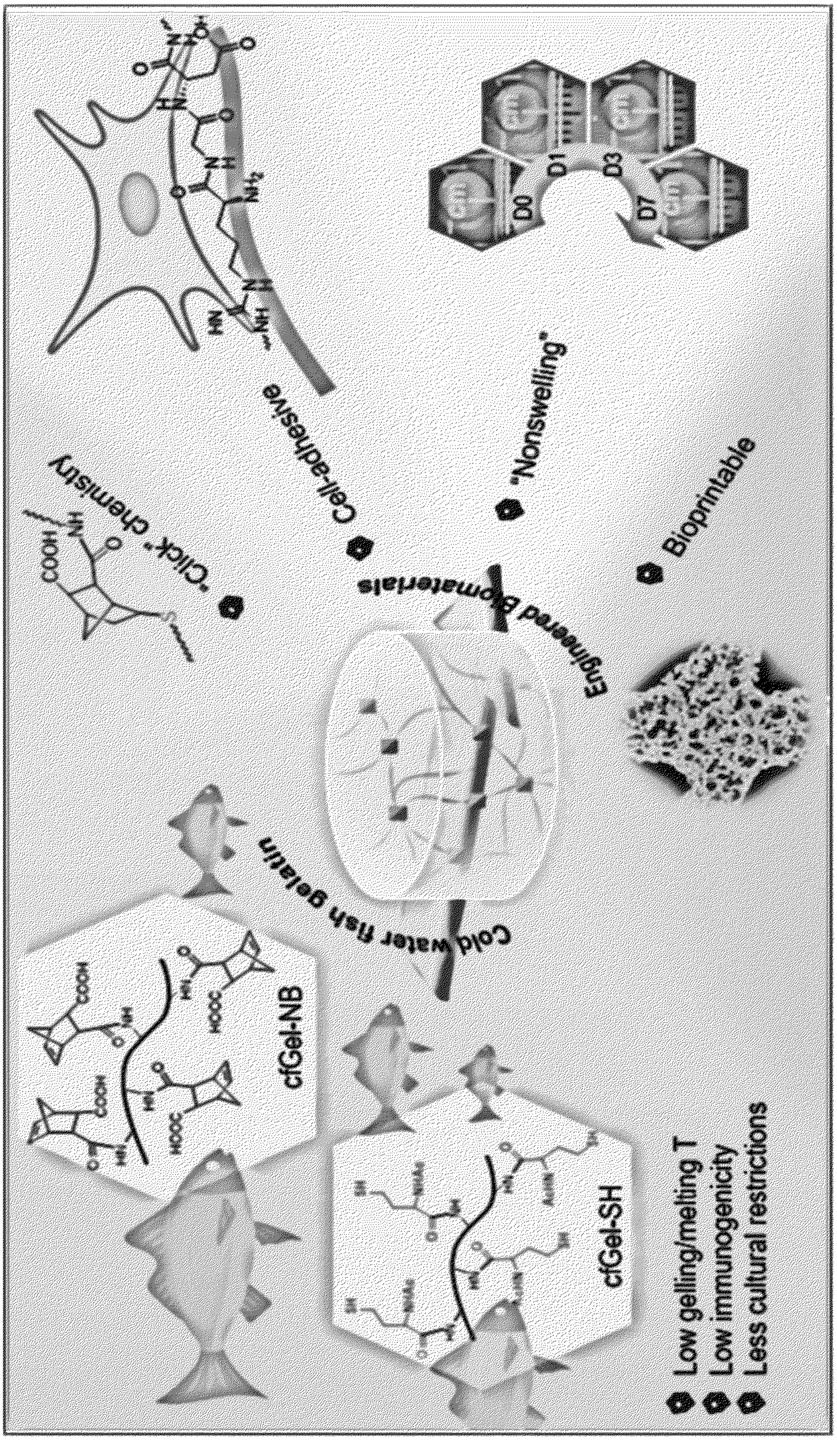
Low gelling/melting T
Low immunogenicity
Less cultural restrictions
cfGel-SH
cfGel-NB
Cold water fish gelatin
Engineered Biomaterials
"Click" chemisty
Cell-adhesive
"Nonswelling"
Bioprintable
D0
D1
D3
D7

Fig. 2

A

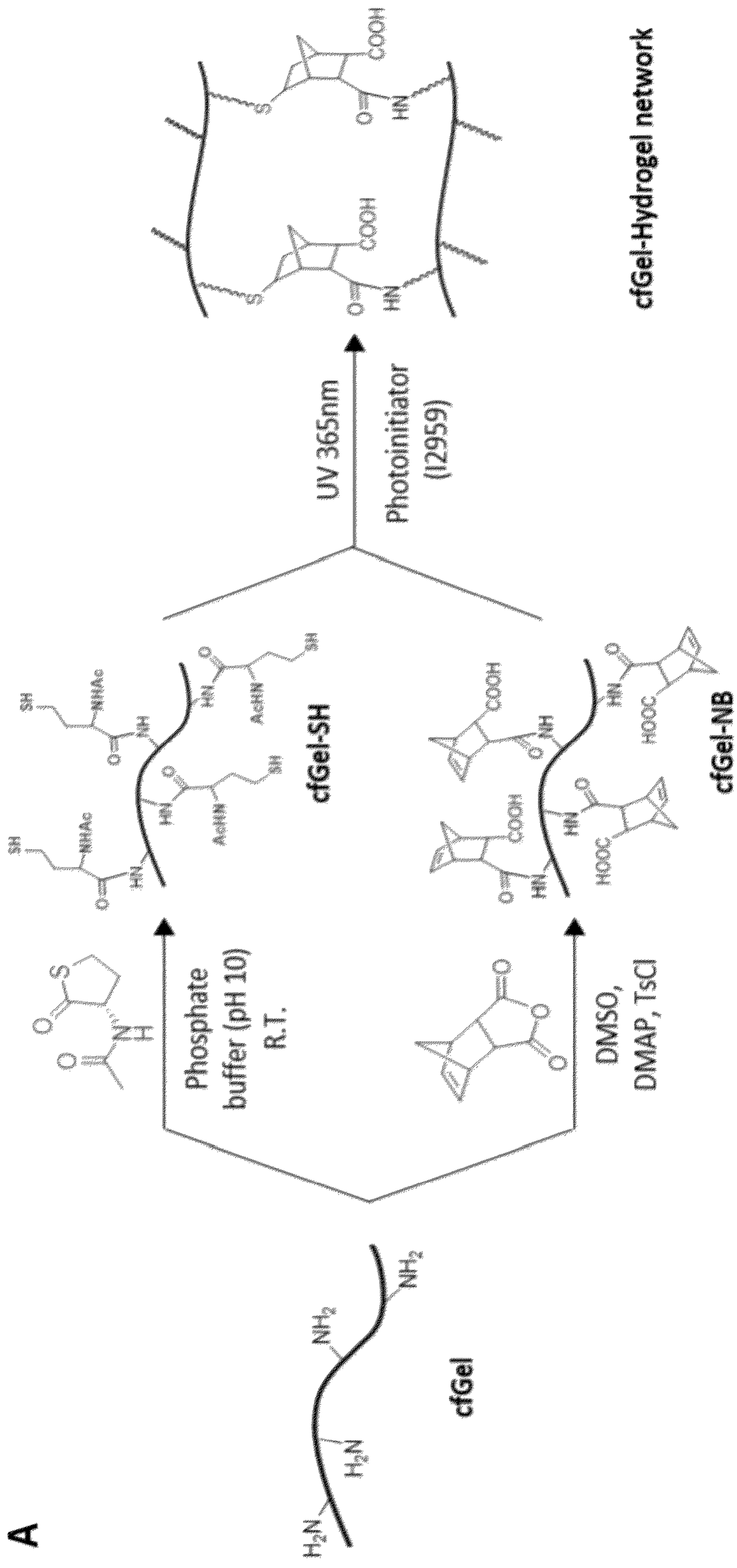
cfGel
Phosphate buffer (pH 10) R.T.
DMSO, DMAP, TsCl
cfGel-SH
cfGel-NB
UV 365nm
Photoinitiator (I2959)
cfGel-Hydrogel network

Fig.2(continues)

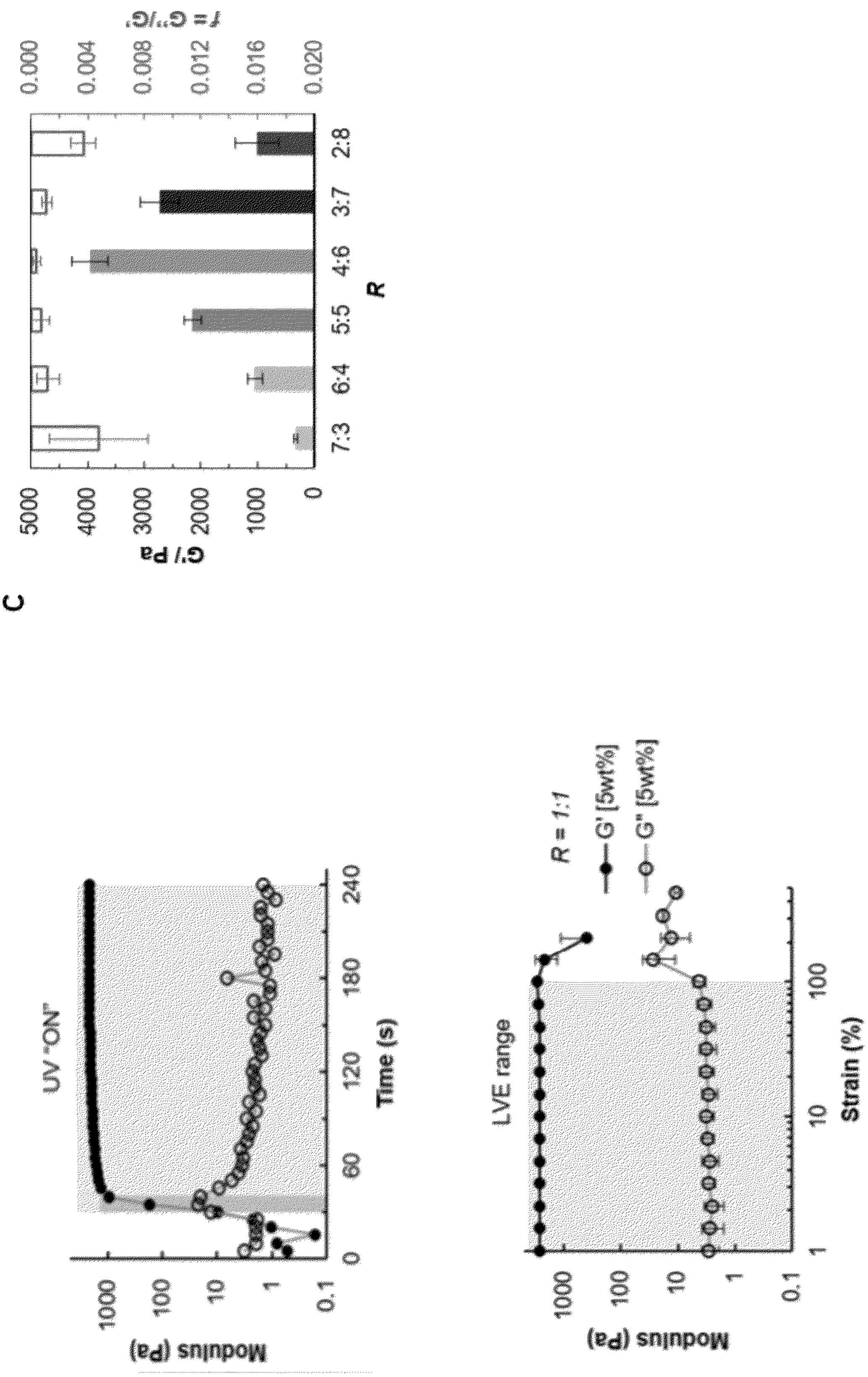

Fig. 3

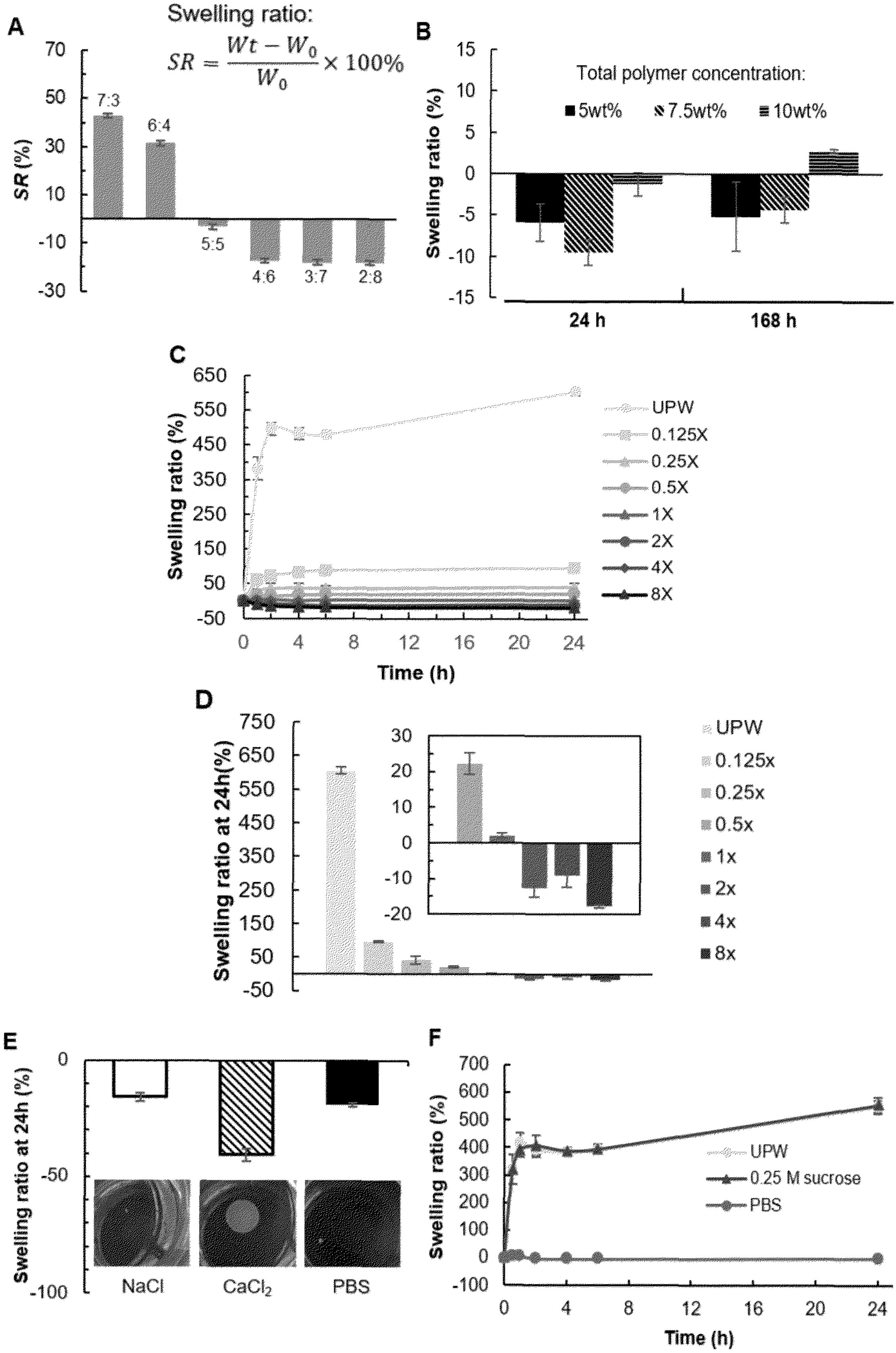
A
Swelling ratio:
$SR = \frac{Wt - W_0}{W_0} \times 100\%$
SR (%)
7:3
6:4
5:5
4:6
3:7
2:8
B
Total polymer concentration:
5wt%
7.5wt%
10wt%
Swelling ratio (%)
24 h
168 h
C
Swelling ratio (%)
UPW
0.125X
0.25X
0.5X
1X
2X
4X
8X
Time (h)
D
Swelling ratio at 24h(%)
UPW
0.125x
0.25x
0.5x
1x
2x
4x
8x
E
Swelling ratio at 24h (%)
NaCl
$CaCl_2$
PBS
F
Swelling ratio (%)
UPW
0.25 M sucrose
PBS
Time (h)

Fig. 4

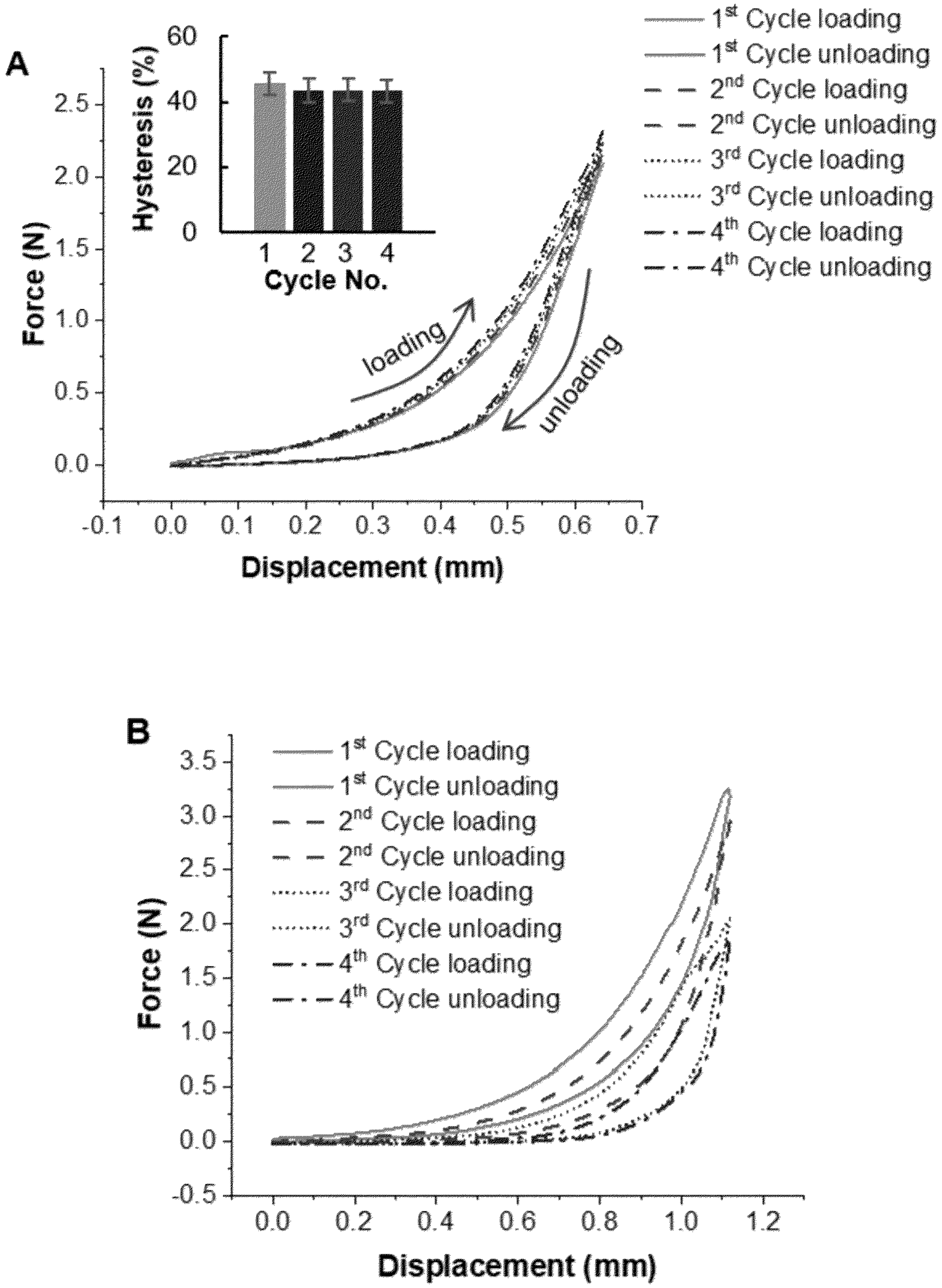
A
Hysteresis (%)
Cycle No.
Force (N)
Displacement (mm)
loading
unloading
1st Cycle loading
1st Cycle unloading
2nd Cycle loading
2nd Cycle unloading
3rd Cycle loading
3rd Cycle unloading
4th Cycle loading
4th Cycle unloading
B
Force (N)
Displacement (mm)
1st Cycle loading
1st Cycle unloading
2nd Cycle loading
2nd Cycle unloading
3rd Cycle loading
3rd Cycle unloading
4th Cycle loading
4th Cycle unloading

Fig. 4 (continues)

C

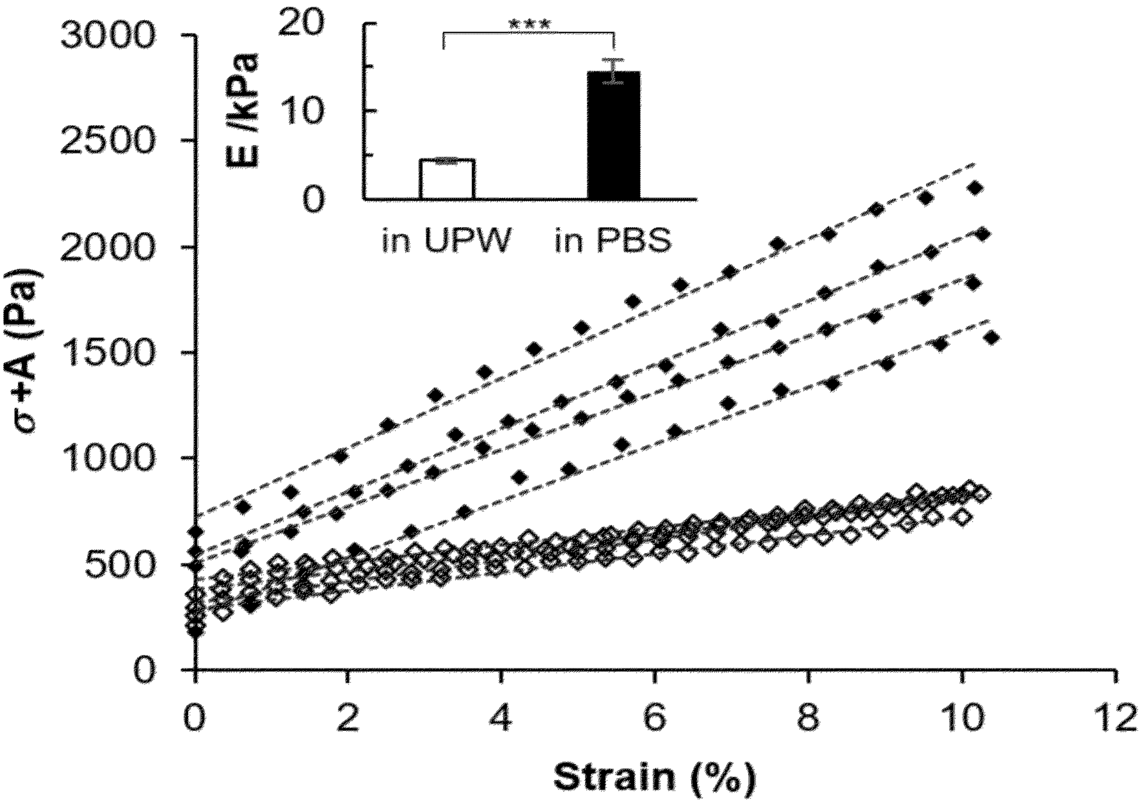


D

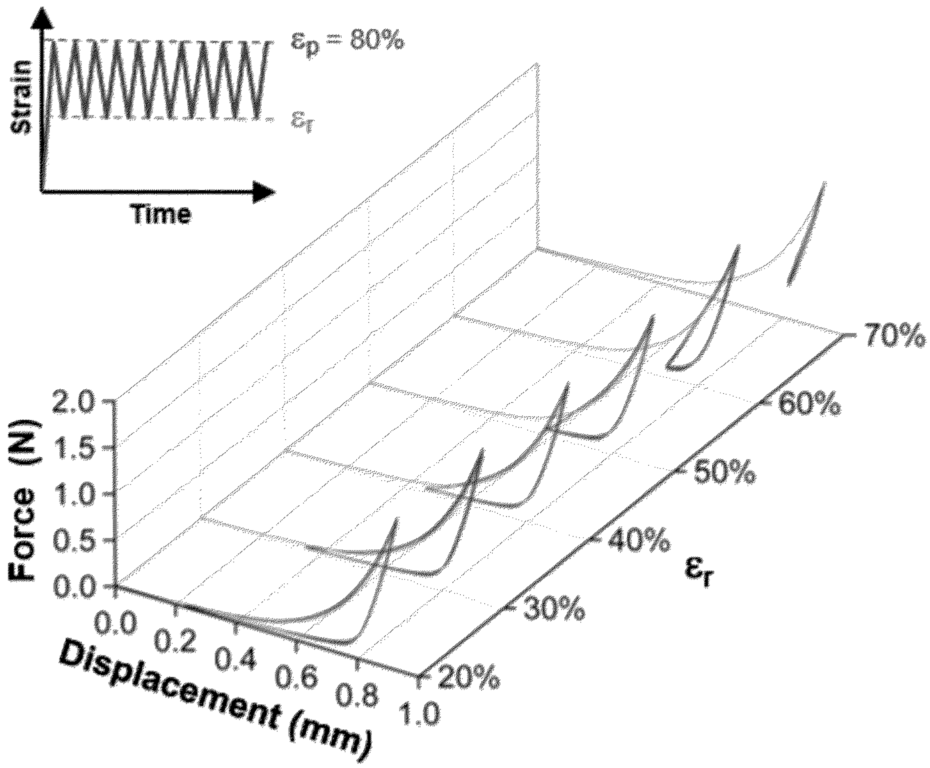

Fig. 5

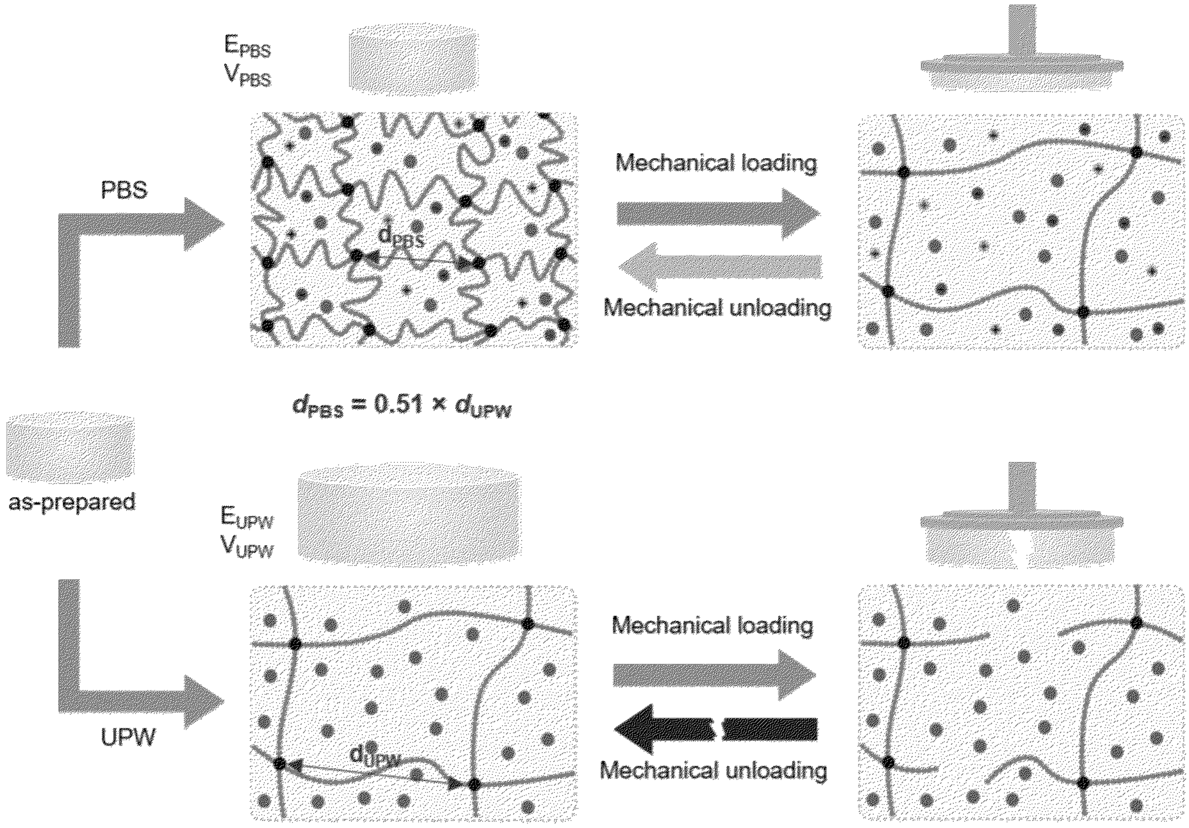
E_PBS
V_PBS
PBS
Mechanical loading
Mechanical unloading
d_PBS
d_PBS = 0.51 × d_UPW
as-prepared
E_UPW
V_UPW
UPW
Mechanical loading
Mechanical unloading
d_UPW

Fig. 6

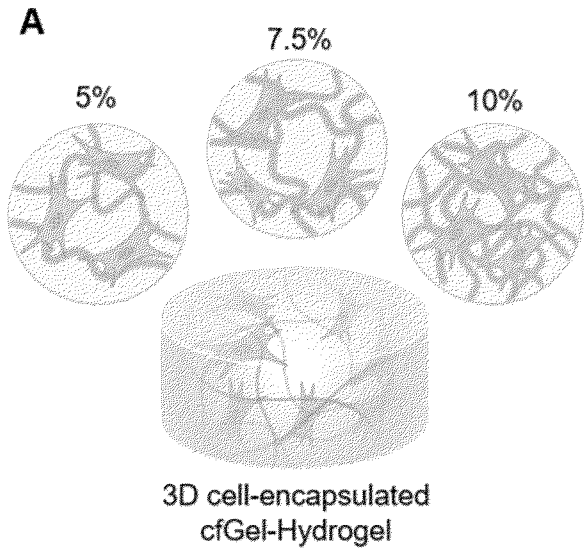
A
7.5%
5%
10%
3D cell-encapsulated
cfGel-Hydrogel

Fig. 6 (continues)

B

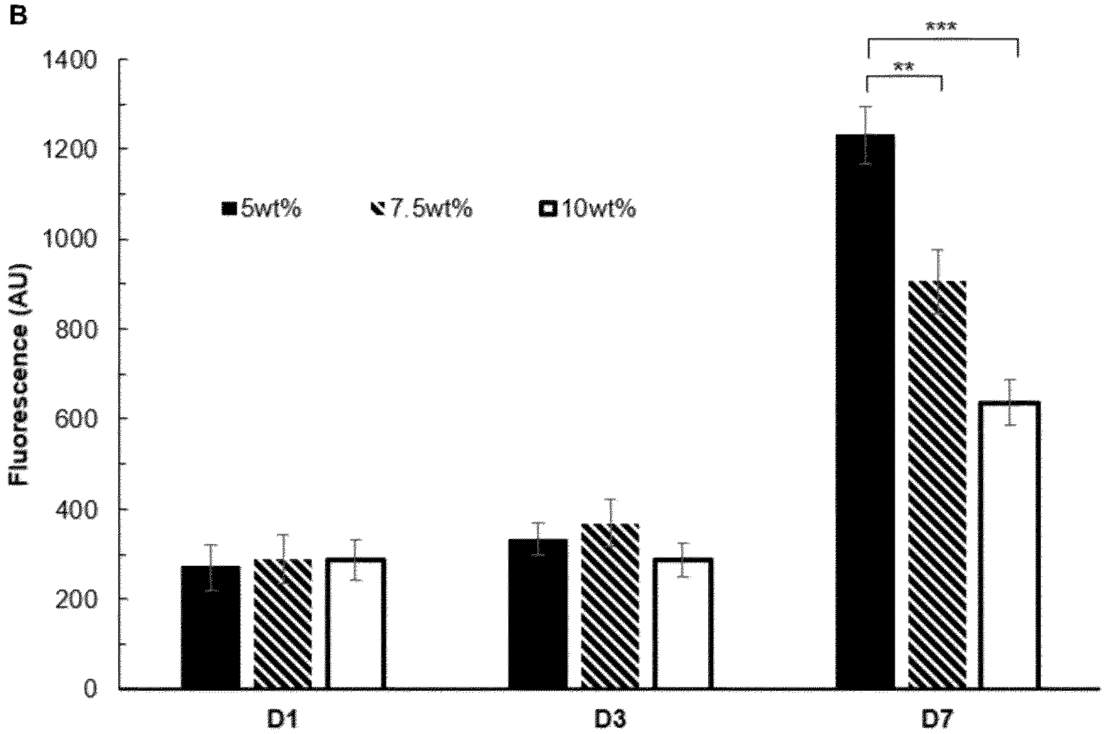


C

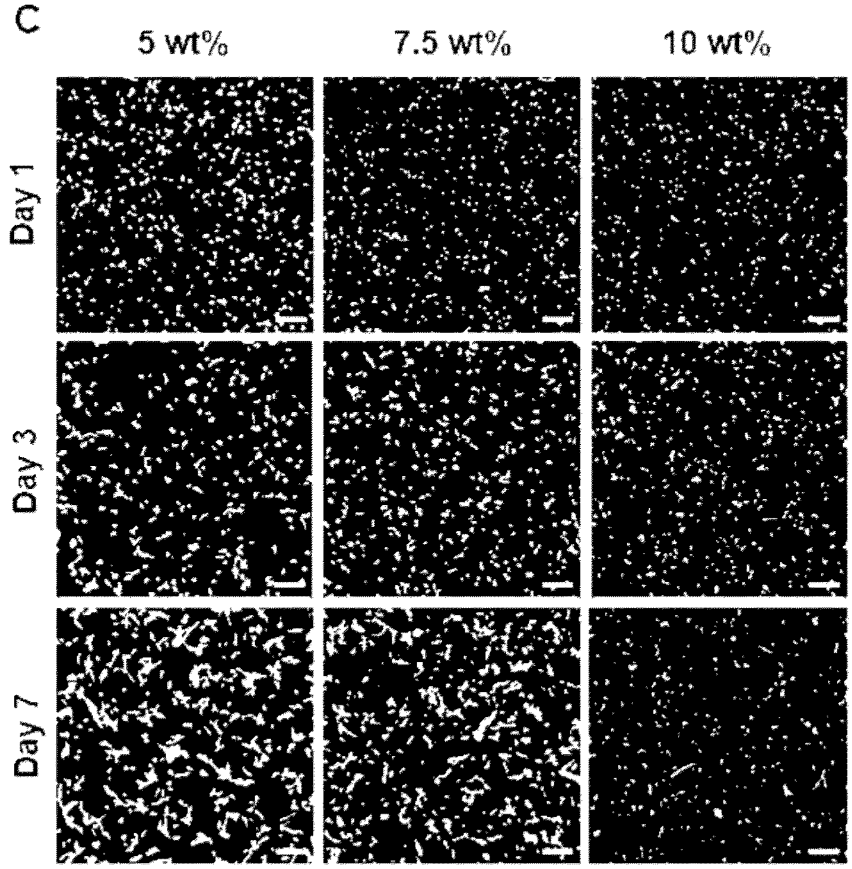

Fig.7

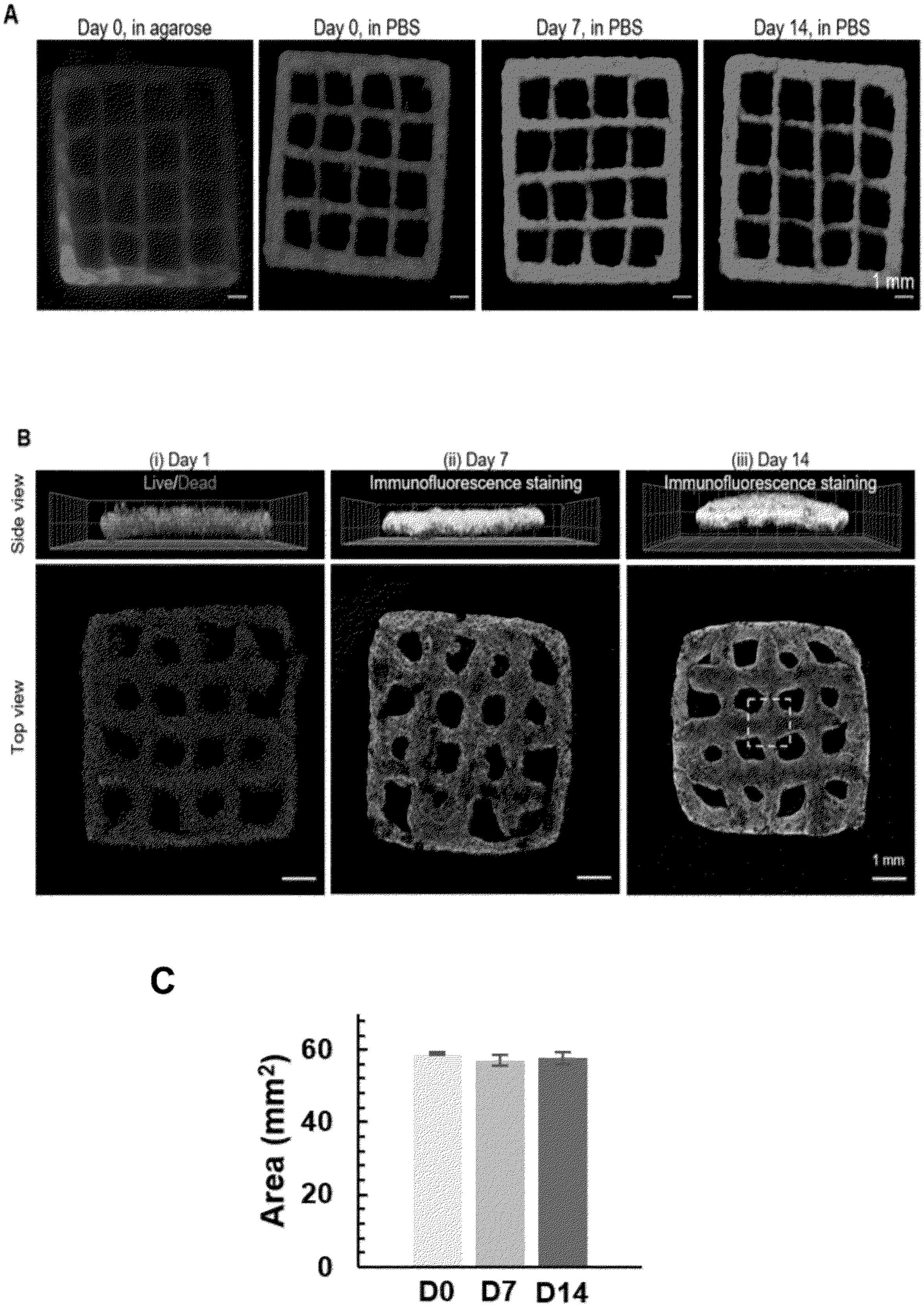
A
Day 0, in agarose
Day 0, in PBS
Day 7, in PBS
Day 14, in PBS
1 mm
B
(i) Day 1
Live/Dead
(ii) Day 7
Immunofluorescence staining
(iii) Day 14
Immunofluorescence staining
Side view
Top view
1 mm
C
Area (mm²)
0
20
40
60
D0
D7
D14

Fig. 8

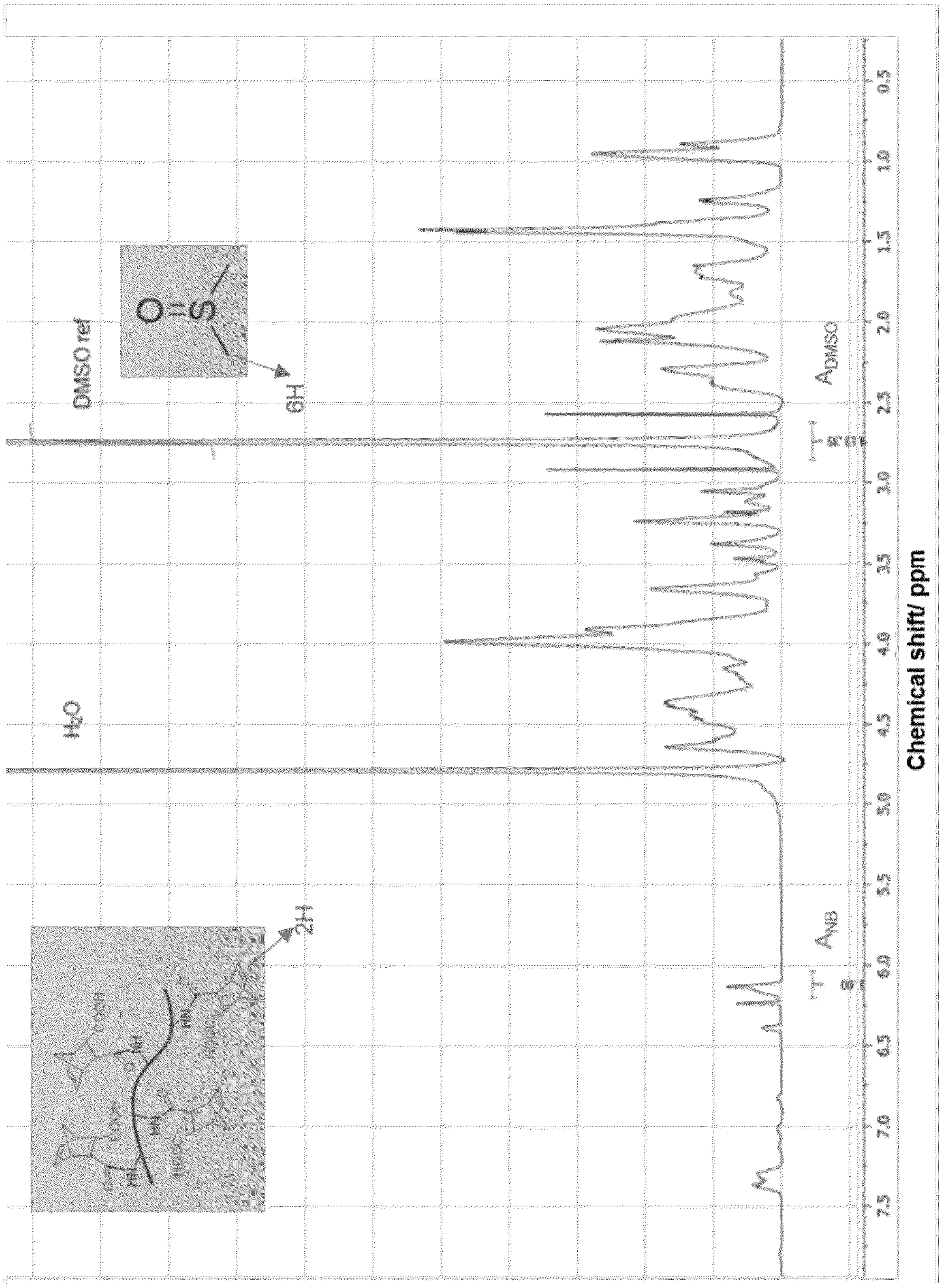
DMSO ref
6H
$H_2O$
2H
$A_{DMSO}$
$A_{NB}$
7.5 7.0 6.5 6.0 5.5 5.0 4.5 4.0 3.5 3.0 2.5 2.0 1.5 1.0 0.5
Chemical shift/ ppm

Fig. 9

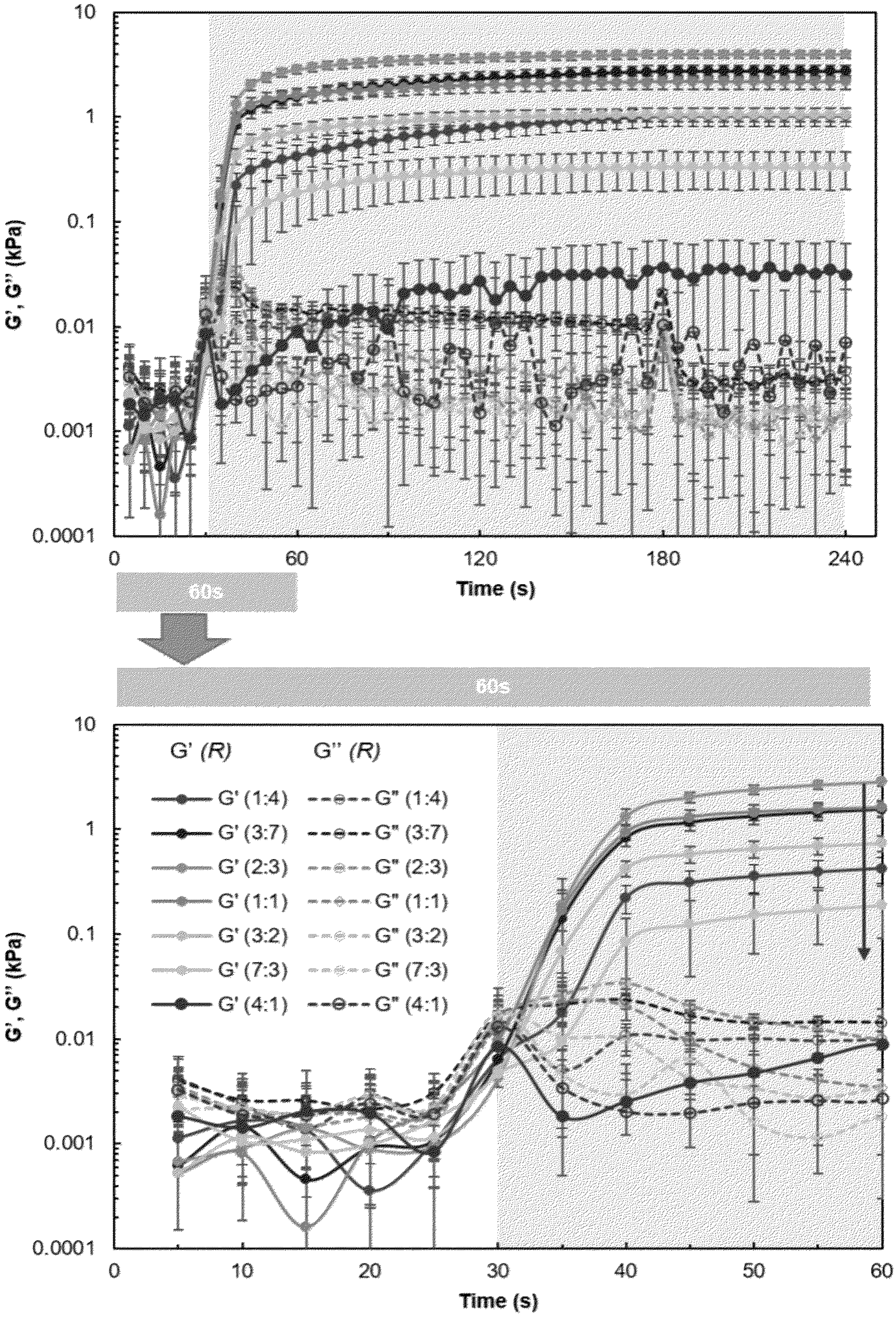
10
1
0.1
0.01
0.001
0.0001
G', G'' (kPa)
0
60
120
180
240
Time (s)
60s
60s
G' (R)
G'' (R)
G' (1:4)
G" (1:4)
G' (3:7)
G" (3:7)
G' (2:3)
G" (2:3)
G' (1:1)
G" (1:1)
G' (3:2)
G" (3:2)
G' (7:3)
G" (7:3)
G' (4:1)
G" (4:1)
10
20
30
40
50
Time (s)

Fig. 10

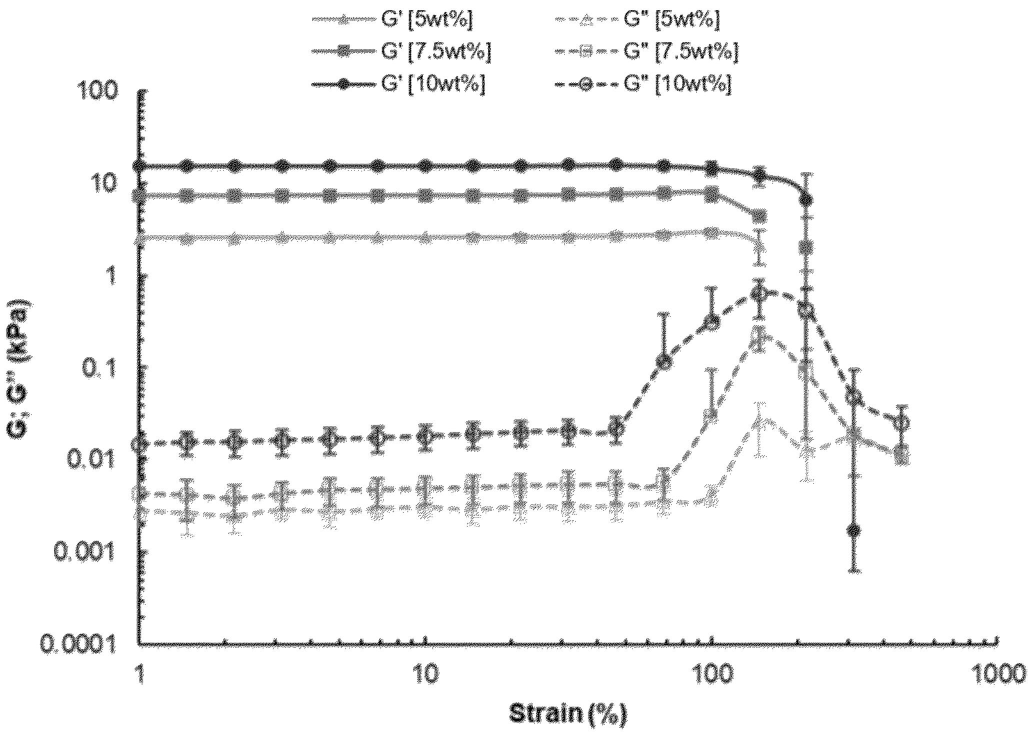
G' [5wt%]
G" [5wt%]
G' [7.5wt%]
G" [7.5wt%]
G' [10wt%]
G" [10wt%]
100
10
1
0.1
0.01
0.001
0.0001
G; G" (kPa)
1
10
100
1000
Strain (%)

Fig. 11

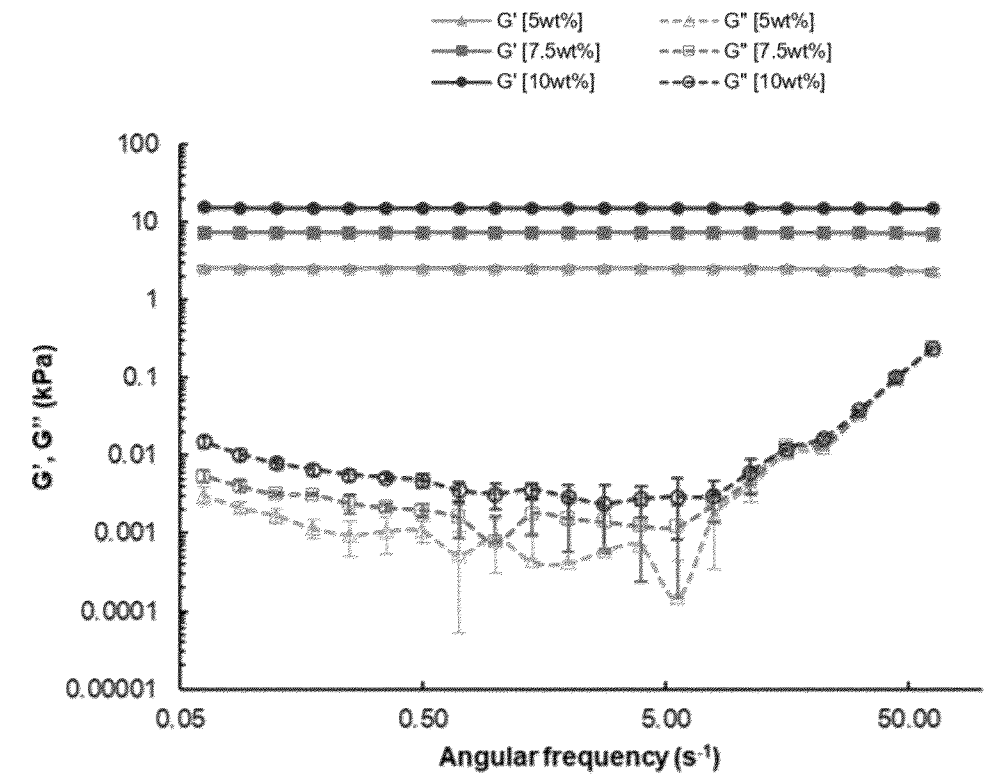
G' [5wt%]
G" [5wt%]
G' [7.5wt%]
G" [7.5wt%]
G' [10wt%]
G" [10wt%]
100
10
1
0.1
0.01
0.001
0.0001
0.00001
G', G" (kPa)
0.05
0.50
5.00
50.00
Angular frequency (s-1)

Fig. 12

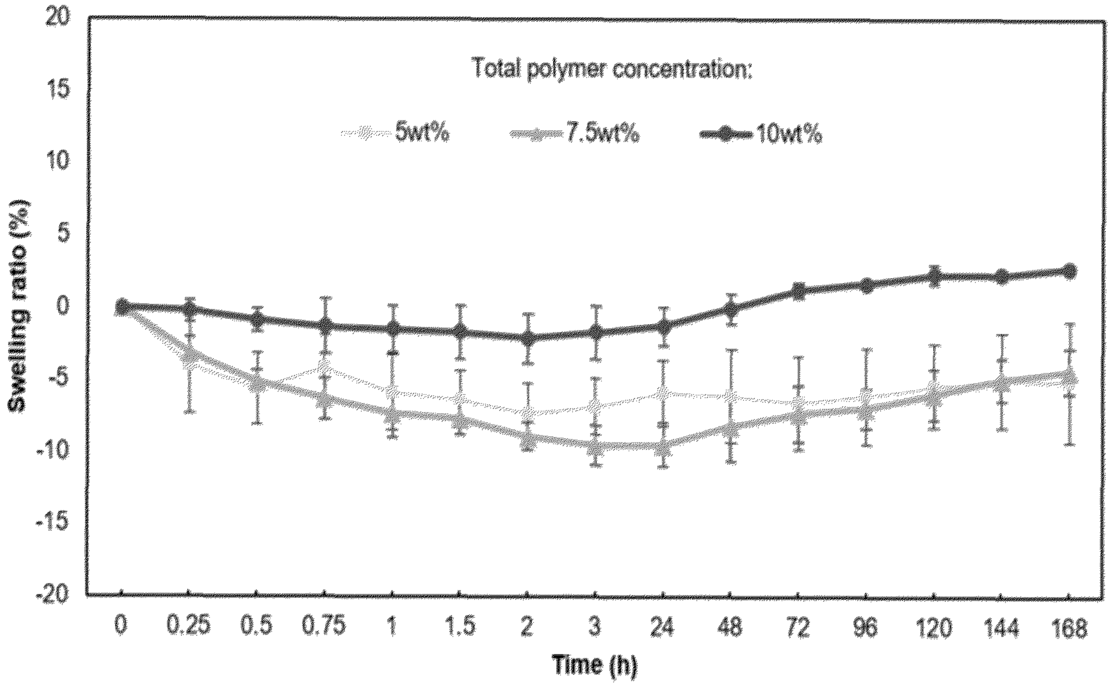
Total polymer concentration:
5wt%
7.5wt%
10wt%
Swelling ratio (%)
20
15
10
5
0
-5
-10
-15
-20
0
0.25
0.5
0.75
1
1.5
2
3
24
48
72
96
120
144
168
Time (h)

Fig. 13

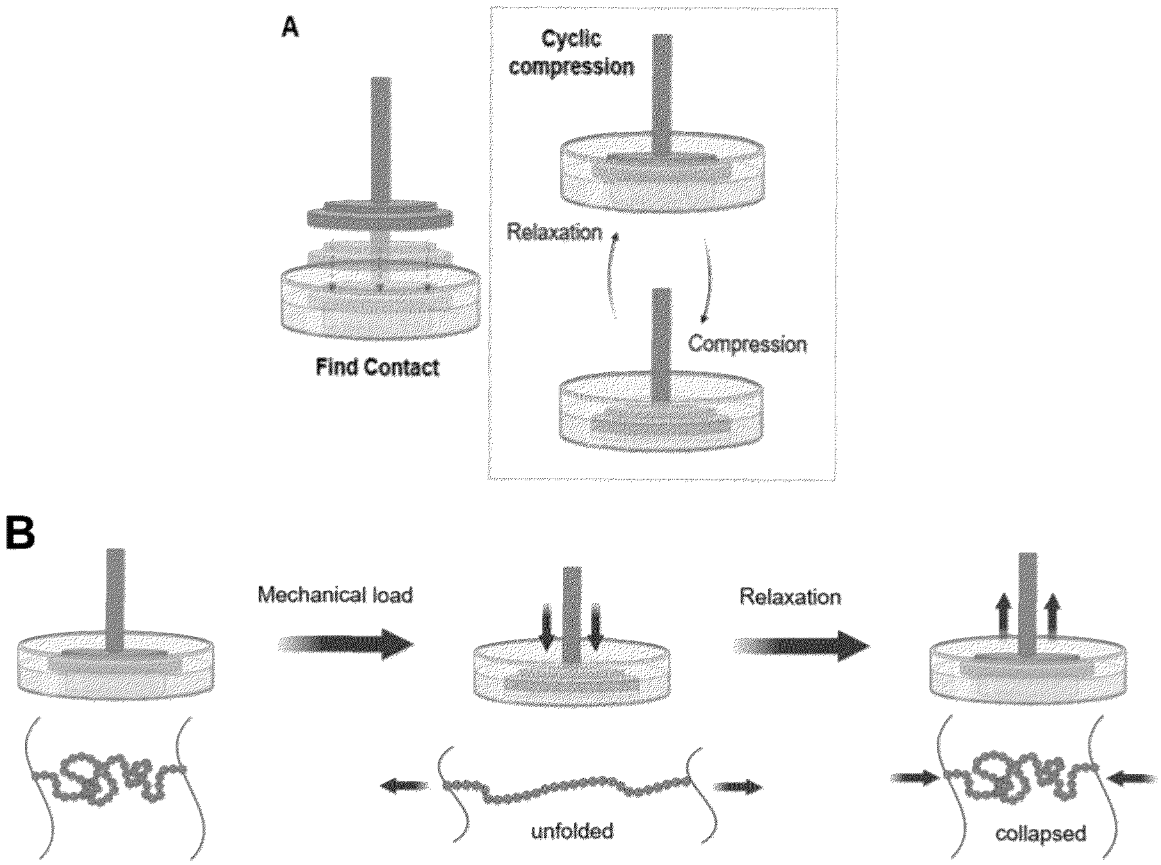
A
Cyclic compression
Relaxation
Compression
Find Contact
B
Mechanical load
Relaxation
unfolded
collapsed

Fig. 14

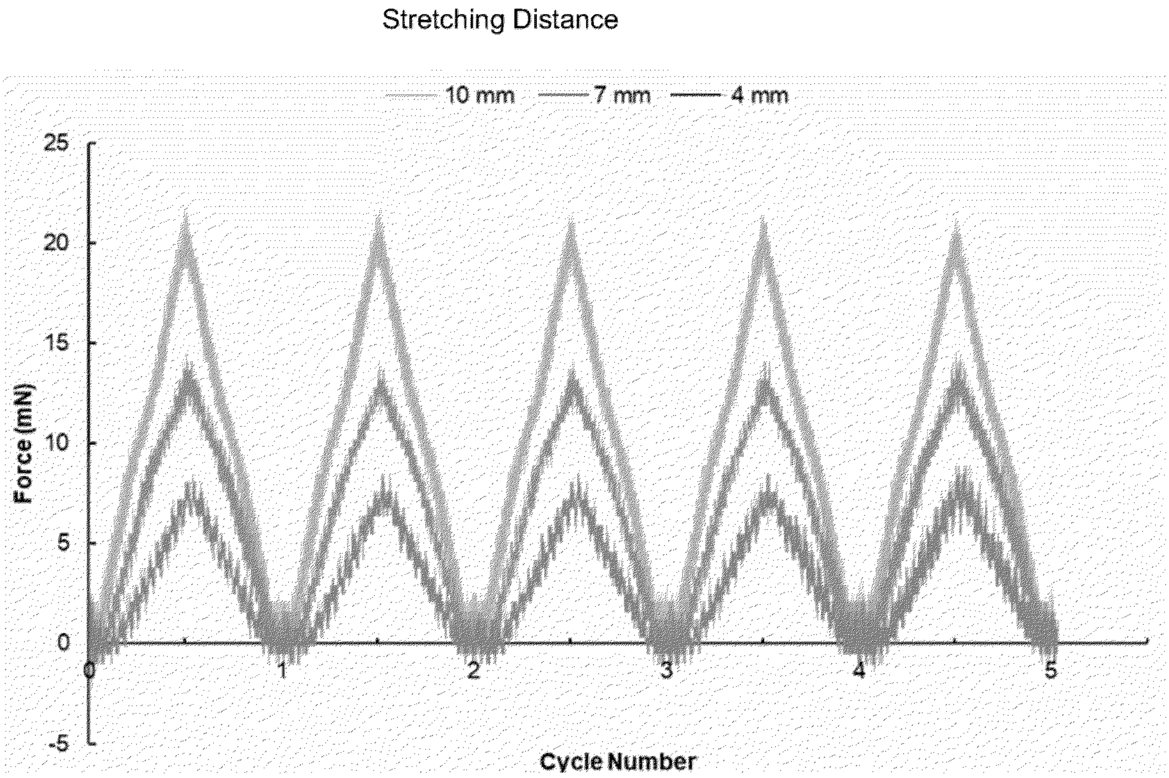
Stretching Distance
10 mm
7 mm
4 mm
Force (mN)
25
20
15
10
5
0
-5
0
1
2
3
4
5
Cycle Number

Fig. 15

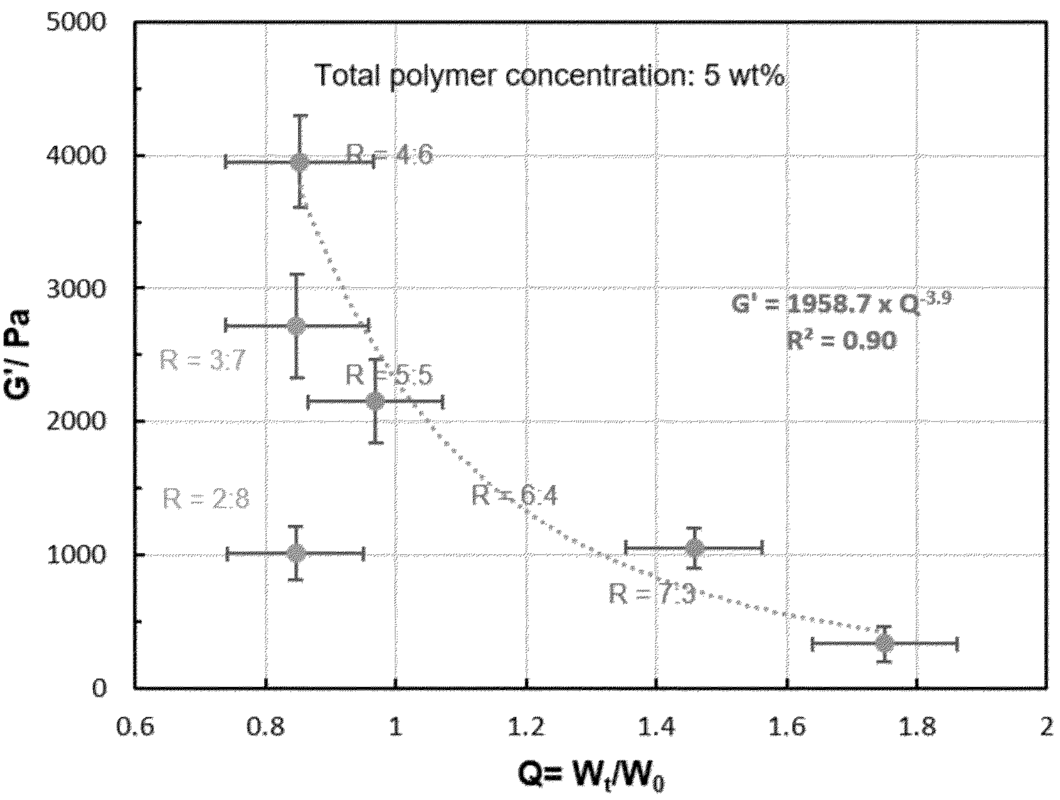
Total polymer concentration: 5 wt%
R = 4:6
R = 3:7
R = 5:5
R = 2:8
R = 6:4
R = 7:3
G' = 1958.7 x Q-3.9
R² = 0.90
G'/ Pa
5000
4000
3000
2000
1000
0
0.6
0.8
1
1.2
1.4
1.6
1.8
2
Q= Wt/W0

Fig. 16

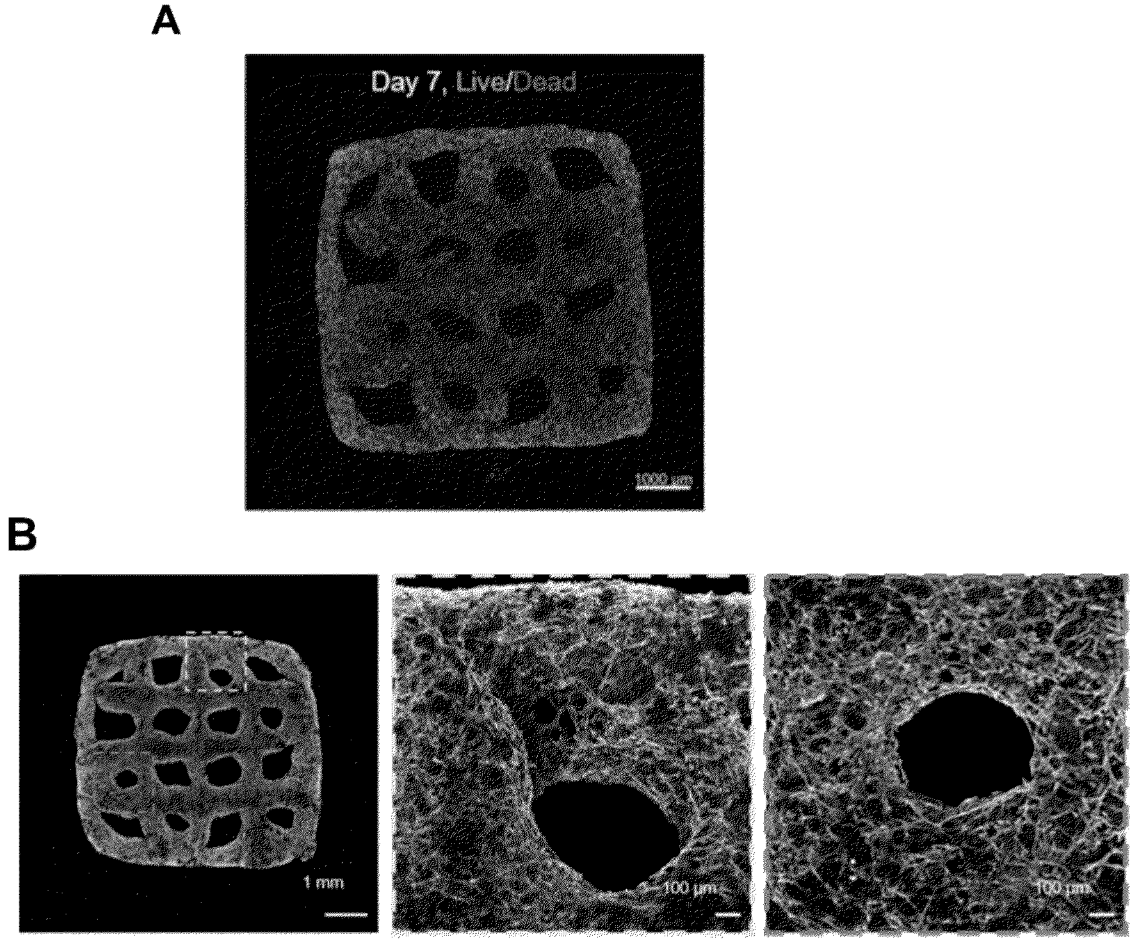
A
Day 7, Live/Dead
1000 µm
B
1 mm
100 µm
100 µm

Fig. 17

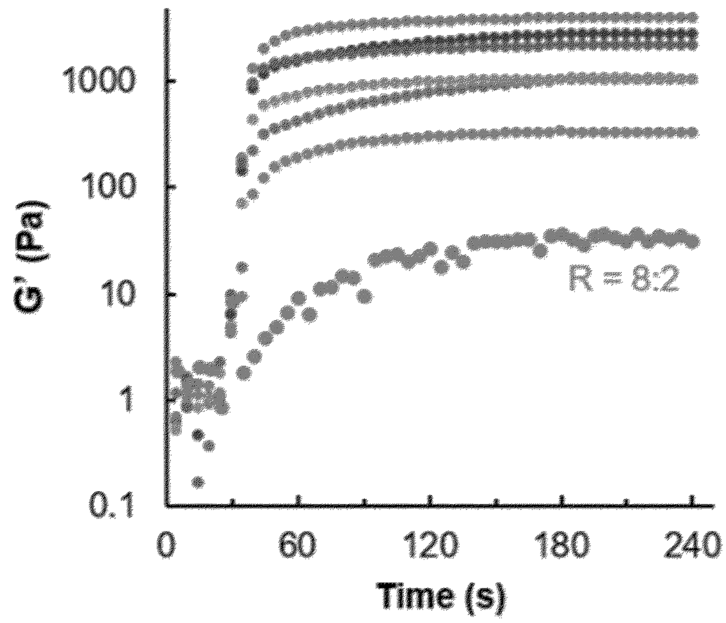
G' (Pa)
1000
100
10
1
0.1
R = 8:2
0
60
120
180
240
Time (s)

Europäisches Patentamt
European Patent Office
Office européen des brevets

# EUROPEAN SEARCH REPORT

Application Number
EP 24 19 4818

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VAN HOORICK JASPER ET AL: "(Photo-)crosslinkable gelatin derivatives for biofabrication applications", ACTA BIOMATERIALIA, ELSEVIER, AMSTERDAM, NL, vol. 97, 22 July 2019 (2019-07-22), pages 46-73, XP085861862, ISSN: 1742-7061, DOI: 10.1016/J.ACTBIO.2019.07.035 [retrieved on 2019-07-22] | 1-5,8-15 | INV. A61L27/22 A61L27/26 A61L27/38 A61L27/52 B33Y70/00 C08L89/06 |
| A | * the whole document * | 6 | |
| | ----- | | |
| A | TOBIAS GÖCKLER ET AL: "Tuning Superfast Curing Thiol-Norbornene-Functionalized Gelatin Hydrogels for 3D Bioprinting", ADVANCED HEALTHCARE MATERIALS, WILEY - V C H VERLAG GMBH & CO. KGAA, DE, vol. 10, no. 14, 19 June 2021 (2021-06-19), page n/a, XP072463023, ISSN: 2192-2640, DOI: 10.1002/ADHM.202100206 * the whole document * | 1-15 | |
| | ----- | | |
| | -/-- | | |

| TECHNICAL FIELDS SEARCHED (IPC) |
|---|
| A61L |
| C12N |
| C09J |
| B33Y |
| C08L |

The present search report has been drawn up for all claims

2

| Place of search | Date of completion of the search | Examiner |
|---|---|---|
| The Hague | 23 January 2025 | Derrien, Anne-Cécile |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 19 4818

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TYTGAT LIESBETH ET AL: "Additive manufacturing of photo-crosslinked gelatin scaffolds for adipose tissue engineering", ACTA BIOMATERIALIA, vol. 94, 1 August 2019 (2019-08-01), pages 340-350, XP093241731, AMSTERDAM, NL ISSN: 1742-7061, DOI: 10.1016/j.actbio.2019.05.062 Retrieved from the Internet: URL:https://pdf.sciencedirectassets.com/273258/1-s2.0-S1742706119X00124/1-s2.0-S1742706119303897/main.pdf?hash=460ba5e6559a34ea448569530a52b95d0fb3dbdf79982a6f3c4fb67743edcbb2&host=68042c943591013ac2b2430a89b270f6af2c76d8dfd086a07176afe7c76c2c61&pii=S1742706119303897&tid=spdf-60782221-b587-4cb5-ac87-516> * the whole document * ----- | 1-15 | |
| A | WO 2024/015568 A2 (UNIV EMORY [US]) 18 January 2024 (2024-01-18) * claims; examples * ----- | 1-15 | TECHNICAL FIELDS SEARCHED (IPC) |
| A | HIPWOOD LUKE ET AL: "Semi-Synthetic Click-Gelatin Hydrogels as Tunable Platforms for 3D Cancer Cell Culture", GELS, vol. 8, no. 12, 12 December 2022 (2022-12-12), page 821, XP093181066, ISSN: 2310-2861, DOI: 10.3390/gels8120821 Retrieved from the Internet: URL:https://www.ncbi.nlm.nih.gov/pmc/articles/PMC9778549/pdf/gels-08-00821.pdf> * the whole document * ----- | 1-15 | |

The present search report has been drawn up for all claims

2

| Place of search | Date of completion of the search | Examiner |
|---|---|---|
| The Hague | 23 January 2025 | Derrien, Anne-Cécile |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT ON EUROPEAN PATENT APPLICATION NO.

EP 24 19 4818

This annex lists the patent family members relating to the patent documents cited in the above-mentioned European search report. The members are as contained in the European Patent Office EDP file on The European Patent Office is in no way liable for these particulars which are merely given for the purpose of information.

23-01-2025

| Patent document cited in search report | Publication date | Patent family member(s) | Publication date |
|---|---|---|---|
| WO 2024015568 A2 | 18-01-2024 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 112220963 B
- WO 2021180795 A1
- WO 2024113021 A1


### Non-patent literature cited in the description


- **MUIR et al.** *Chemical reviews*, 2020, vol. 121 (18), 10908-10949
- **YOON et al.** *PloS one*, 2016, vol. 11 (10), e0163902
- **BELLIS**. *Biomaterials*, 2011, vol. 32 (18), 4205-4210
- **LE THI et al.** *Journal of Biomaterials Applications*, 2020, vol. 34 (9), 1216-1226
- **NITSUWAT et al.** *Lwt*, 2021, vol. 141, 110899
- **ZHAN et al.** *Materials Science and Engineering: C*, 2021, vol. 127, 112208
- **SAMBROOK et al.** Molecular Cloning: A Laboratory Manual. Cold Spring Harbor Laboratory Press, 2012
- **AUSUBEL et al.** Short Protocols in Molecular Biology. John Wiley & Sons, Inc., 2002
- *CHEMICAL ABSTRACTS*, 61551-69-7
- **BENEDIKT et al.** *J. Polymer Science Part A Polymer Chemistry*, 2015, vol. 54, 473-479, https://doi.orq/10.1002/pola.27903
- **TROMAYER et al.** *Polymer Chemistry*, 2018, vol. 9, 3108-3117, https://doi.org/10.1039/C8PY00278A
- **SHIWARSKI et al.** *APL Bioeng.*, March 2021, vol. 5 (1), 010904
- **JASPER VAN HOORICK et al.** *Biofabrication*, 2021, vol. 13, 015017
- **SCHWAB, A. et al.** *Chem Rev*, 2020, vol. 120 (19), 11028
- **PRENDERGAST, M. E et al.** *Adv Healthc Mater.*, 2021, e2101679
- **MUIR, V. G.** ; **BURDICK, J. A. et al.** Biopolymers for the Formation of Biomedical Hydrogels. *Chem Rev*, 2021, vol. 121 (18), 10908-10949
- **YOON, H. J. et al.** Cold Water Fish Gelatine Methacryloyl Hydrogel for Tissue Engineering Application. *PLoS One*, 2016, vol. 11 (10), e0163902
- **BELLIS, S. L. et al.** Advantages of RGD peptides for directing cell association with biomaterials. *Biomaterials*, vol. 32 (18), 4205
- **LE THI P et al.** Horseradish peroxidase-catalyzed hydrogelation of fish gelatine with tunable mechanical properties and biocompatibility. *Journal of Biomaterials Applications*, 2020, vol. 34 (9), 1216-1226
- **NITSUWAT, S. et al.** Fish gelatin as an alternative to mammalian gelatin for food industry: A meta-analysis. *LWT*, 2021, vol. 141, 110899
- **ZHAN, Y. et al.** Advances in versatile anti-swelling polymer hydrogels. *Mater Sci Eng C Mater Biol Appl*, August 2021, vol. 127, 112208
- **JASPER VAN HOORICK et al.** Thiol-norbornene gelatin hydrogels: influence of thiolated crosslinker on network properties and high definition 3D printing. *Biofabrication*, 2021, vol. 13, 015017
- **SCHWAB A et al.** Printability and Shape Fidelity of Bioinks in 3D Bioprinting. *Chem Rev*, 2020, vol. 120 (19), 11028-11055
- **LEE, A. et al.** 3D bioprinting of collagen to rebuild components of the human heart. *Science*, vol. 365 (6452), 482-487
- **PRENDERGAST ME**. Computational Modeling and Experimental Characterization of Extrusion Printing into Suspension Baths.. *Adv Healthc Mater.*, April 2022, vol. 11 (7), e2101679